Europäisches Patentamt

European Patent Office

Office européen des brevets

(19)

(11) **EP 0 861 044 B1**

(12)     **EUROPÄISCHE PATENTSCHRIFT**

(45) Veröffentlichungstag und Bekanntmachung des Hinweises auf die Patenterteilung:
**07.03.2001  Patentblatt 2001/10**

(51) Int Cl.[7]: **A61B 5/00**

(21) Anmeldenummer: **96943007.3**

(86) Internationale Anmeldenummer:
**PCT/DE96/01831**

(22) Anmeldetag: **26.09.1996**

(87) Internationale Veröffentlichungsnummer:
**WO 97/11636 (03.04.1997 Gazette 1997/15)**

(54) **VORRICHTUNG ZUR PHOTODYNAMISCHEN DIAGNOSE**

APPARATUS FOR PHOTODYNAMIC DIAGNOSIS

DISPOSITIF DE DIAGNOSTIC PHOTODYNAMIQUE

(84) Benannte Vertragsstaaten:
**DE FR GB IT**

(30) Priorität: **26.09.1995  DE 19535829**
**31.12.1995  DE 19548913**

(43) Veröffentlichungstag der Anmeldung:
**02.09.1998  Patentblatt 1998/36**

(73) Patentinhaber: **Karl Storz GmbH & Co. KG**
**78532 Tuttlingen (DE)**

(72) Erfinder:
 • **IRION, Klaus**
   **D-78576 Emmingen-Liptingen (DE)**
 • **BAUMGARTNER, Reinhold**
   **D-85354 Freising (DE)**
 • **STEPP, Herbert**
   **D-82152 Planegg (DE)**

 • **EHRHARDT, André**
   **D-78532 Tuttlingen (DE)**
 • **STROBL, Karlheinz**
   **Fiskdale, MA 01518 (US)**

(74) Vertreter: **Münich, Wilhelm, Dr.**
**Dr. Münich & Kollegen**
**Anwaltskanzlei**
**Wilhelm-Mayr-Str. 11**
**80689 München (DE)**

(56) Entgegenhaltungen:
**EP-A- 0 241 268       EP-A- 0 339 582**
**WO-A-86/02730        WO-A-93/13403**
**DE-A- 4 133 493      GB-A- 2 126 717**
**GB-A- 2 258 728      US-A- 4 056 724**
**US-A- 5 371 624      US-A- 5 450 857**

Anmerkung: Innerhalb von neun Monaten nach der Bekanntmachung des Hinweises auf die Erteilung des europäischen Patents  kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

**Technisches Gebiet**

Die Erfindung bezieht sich auf eine Vorrichtung zur "in vivo-Diagnose" mittels einer durch einen körpereigenen oder körperfremden Photosensibilisator lichtinduzierten Reaktion in biologischem Gewebe.

**Stand der Technik**

Aus der US-A-5 371 624 ist eine Vorrichtung, bekannt bei der der Reintransmissionsgrad des aus licht zuführender und aus bildgebender Einheit bestehenden Gesamtsystems im gesamten Wellenlängenbereich vernachlässigbar gering ist. Das mit einer derartigen Vorrichtung ausgeführte Diagnose-Verfahren wird in der medizinischen Fachsprache auch als photodynamische Diagnose (PDD) oder als Fluoreszenz-Diagnose bezeichnet. Ferner ist bekannt, Photosensibilisatoren zur photodynamischen Therapie (PDT) einzusetzen. Hierzu wird auf die WO 93/20810 verwiesen, auf die im übrigen hinsichtlich der Erläuterung aller hier nicht näher beschriebenen Begriffe und Verfahrensschritte ausdrücklich Bezug genommen wird.

Es ist ferner vorgeschlagen worden, eine endoskopische photodynamische Diagnose und Therapie mit einer Vorrichtung auszuführen, bei der als Lichtquelle ein Kryptonionenlaser mit einer Wellenlänge von ca. 410 nm und einer Leistung von mehr als 200 mWatt verwendet wird. Das Licht dieses Lasers wird über einen Quarzlichtleiter mit geringer numerischer Apertur durch ein Endoskop an die zu bestrahlenden Stellen des menschlichen Körpers geleitet.

Um eine lichtinduzierte Reaktion in biologischen Systemen auszulösen, wird dem Patienten ein Photosensibilisator, der entweder ein Hämatoporphyrin-Grundgerüst wie die Substanzen Photofrin und Photosan-3 aufweist, oder Delta-Aminolävulinsäure (ALA), die seit kurzem in der Urologie und Dermatologie Verwendung findet, in einer Konzentration von wenigen mg/kg Körpergewicht verabreicht. Die Hämatoporphyrin-Derivate werden intravenös verabreicht, Delta-Aminolävulinsäure kann hingegen lokal appliziert werden, d.h. sie wird als Lösung beispielsweise in die Harnblase injiziert. Diese Substanzen reichern sich nun in Tumorgeweben in 2 bis 10-fach erhöhter Konzentration an. Diese selektive Anreicherung im Tumorgewebe stellt die entscheidende Grundlage für die photodynamische Diagnose und die photodynamische Therapie dar.

Zur Diagnose wird das zu untersuchende Gewebe ca. 2-12 Stunden nach Verabreichung des Photosensibilisators (ALA) endoskopisch mit violettem Licht - bei bekannten Vorrichtungen nahezu ausschließlich Laserlicht - bestrahlt. Die Porphyrinderivate, die im Tumorgewebe in einer erhöhten Konzentration vorliegen, werden durch dieses Licht angeregt und weisen anschließend eine typische Rotfluoreszenz auf, durch die der Tumor lokalisiert werden kann.

Neben der Fluoreszenz - bewirkt durch einen im Gewebe angereicherten Photosensibilisator- kann auch die sogenannte Autofluoreszenz des Gewebes ausgelöst werden, die durch sogenannte Fluorophoren, d.h. körpereigene Fluoreszenzstoffe zustande kommt.

Bei der photodynamischen Therapie wird eine Bestrahlung mit rotem Laserlicht durchgeführt, da dieses Licht mit einer Wellenlänge von mehr als 630 nm eine Eindringtiefe in das Gewebe von ca. 5 mm erreicht, im Gegensatz zu Licht kürzerer Wellenlängen, das eine eklatant niedrigere Eindringtiefe besitzt. Trotz Verwendung dieser optimalen Wellenlänge ist die Indikation für die photodynamische Therapie derzeit auf flache oberflächlich gelegene Karzinome beschränkt.

Der biophysikalische Ablauf der lichtinduzierten Reaktion kann wie folgt angenommen werden:

Der im Gewebe eingelagerte Photosensibilisator wird durch die Absorption eines Lichtquants mit definiertem Energiegehalt, das von der jeweiligen Lichtquelle ausgesandt wird, in einen angeregten Zustand überführt. Bei der Bestrahlung mit violettem Licht im Rahmen der photodynamischen Diagnose wird nun beim Zurückfallen in den Grundzustand Fluoreszenzstrahlung emittiert.

Im Falle der photodynamischen Therapie in Verbindung mit der Bestrahlung mit rotem Laserlicht hoher Leistungsdichte findet der Übergang von der angeregten Form in einen metastabilen Zwischenzustand statt, von dem aus die Energie, die durch Rückgang in den Grundzustand frei wird, auf molekularen Sauerstoff übertragen wird, der diese Energie unter Bildung von angeregtem Singulett-Sauerstoff aufnimmt. Dieser aggressive Singulett-Sauerstoff zerstört im betreffenden Gewebe durch Photooxidation Zellstrukturen. Diese zellulären Schäden führen zusammen mit einem gleichzeitig eintretenden Zusammenbruch des tumoralen Gefäßsystems zu einer kompletten Tumorzerstörung (phototoxischer Effekt).

Dieses Verfahren ist jedoch in Abhängigkeit von den verwendeten Photosensibilisatoren mit gewissen Problemen behaftet. Bei dem Einsatz von Photofrin und Photosan-3 als Photosensibilisatoren bei der photodynamischen Diagnose müssen für den Fluoreszenznachweis sehr aufwendige technische Vorrichtungen verwendet werden, da durch störende Eigenfluoreszenzanteile nur mit Hilfe sehr aufwendiger computergestützter Bildverarbeitungstechniken und hochempfindlichen Kameras mit Restlichtverstärker die Fluoreszenz des Tumorgewebes entsprechend nachgewiesen werden kann.

Bei der Verwendung von Delta-Aminolävulinsäure ist die induzierte Fluoreszenz stark genug, daß sie rein visuell erkannt werden kann. Aber auch die durch Delta-Aminolävulinsäure erreichte Fluoreszenz führt nicht zu einer optimalen Qualität des endoskopischen Bildes, das im Rahmen der Diagnose aufgezeichnet werden soll. Der Einsatz eines Quarzlichtleiters mit geringer numerischer Apertur, wie er für die Lichtübertragung eines Laserstrahls verwendet wird, ermöglicht nur eine sehr schlechte Ausleuchtung des Bildes.

Durch die Verwendung eines zusätzlichen Lichtleiters, der die Ausleuchtung verbessern könnte, wird das für weitere Kanäle im Endoskop zur Verfügung stehende Lumen derart reduziert, daß die Verwendung anderer endoskopischer Instrumente stark eingeschränkt wird.

Vor allem aber überstrahlt bei Verwendung bekannter Vorrichtungen eventuell in das Endoskop eingeleitetes zusätzliches Beleuchtungslicht das auch bei Verwendung von Delta-Aminolävulinsäure vergleichsweise schwache Fluoreszenzbild.

Darüberhinaus sind bei oben beschriebenen Verfahren für die Diagnose und Therapie unterschiedliche Licht bzw. Laserquellen notwendig, was einerseits die Kosten ansteigen läßt, und andererseits die Handhabung des endoskopischen Systems erschwert.

Ähnliche Probleme treten auch bei der Durchführung der photodynamischen Diagnose mittels eines Mikroskops und insbesondere eines Operationsmikroskops auf.

**Darstellung der Erfindung**

Der Erfindung liegt die Aufgabe zugrunde, eine Vorrichtung zur Diagnose mittels einer durch einen körperfremden oder körpereigenen Photosensibilisator lichtinduzierten oder durch Eigenfluoreszenz hervorgerufenen Reaktion in biologischem Gewebe derart weiterzubilden, daß die induzierte Fluoreszenz bei gleichzeitiger für eine Beobachtung des Gewebebereichs ausreichender Ausleuchtung - bevorzugt ohne den Einsatz von Lasern - kontraststark erkannt werden kann.

Zwei bevorzugt alternativ einsetzbare erfindungsgemäße Lösungen dieser Aufgabe sind in den Patentansprüchen 1 bzw. 2 angegeben. Weiterbildungen der Erfindung sind Gegenstand der Ansprüche 3 folgende.

Den in den beiden Hauptansprüchen angegebenen Lösungen der erfindungsgemäß gestellten Aufgabe liegt folgender einheitlicher Grundgedanke zugrunde:

Die Reintransmissionsgrade im lichtzuführenden und im bilderzeugenden Teil der erfindungsgemäßen Vorrichtung sind so gewählt, daß zum einen praktisch kein Licht mit einer Wellenlänge $\lambda$ aus dem zur Anregung benutzten Bereich, das naturgemäß eine vergleichsweise hohe Intensität hat, durch den bilderzeugenden Teil der Vorrichtung in die proximale Bildebene "gelangt", während Licht mit einer Wellenlänge $\lambda$ aus dem Bereich, in dem Fluoreszenz auftritt, nur dann in die proximale Bildebene gelangen kann, wenn es aus dem beleuchteten Gewebebereich und nicht aus dem Beleuchtungssytem kommt. Zum anderen gewährleisten die Reintransmissionsgrade des lichtzuführenden und des bilderzeugenden Teils der Vorrichtung, daß der beleuchtete Gewebebereich so stark mit Licht mit einer Wellenlänge beleuchtet wird, die weder im Bereich des Anregungsspektrums noch im Bereich des Fluoreszenzspektrums liegt, daß die Untersuchungsperson aufgrund des in diesem Wellenlängenbereich direkt reflektierten Lichts Einzelheiten des beleuchteten Gewebebereichs unabhängig von der Fluoreszenzstrahlung wahrnehmen kann.

Anders ausgedrückt, wird erfindungsgemäß das Bild des mit Anregungslicht beleuchteten Gewebebereichs gleichzeitig mittels Fluoreszenzlicht und reflektierten Beleuchtungslicht erzeugt, wobei die beiden zur Bilderzeugung beitragenden Anteile bezüglich ihrer Wellenlänge und bezüglich ihrer Intensität so beschaffen sind, daß sie sich nicht gegenseitig "stören".

Die erfindungsgemäße Vorrichtung zur Diagnose mittels einer durch einen körperfremden oder körpereigenen Photosensibilisator bzw. durch Eigenfluoreszenz lichtinduzierten Reaktion in biologischem Gewebe weist ein Beleuchtungssystem mit mindestens einer Lichtquelle, eine lichtzuführende Einheit, die das Licht des Beleuchtungssystems auf den zu diagnostizierenden Gewebebereich richtet, und eine bildgebende Einheit auf, die das von dem Gewebebereich kommende Licht in eine proximale Bildebene abbildet. Vorrichtungen zur Diagnose von Körpergewebe mit den vorgenannten Merkmalen sind allgemein bekannt und werden beispielsweise in der Endoskopie oder Mikroskopie eingesetzt.

Erfindungsgemäß wird bei der im Anspruch 1 und bevorzugt auch bei der im Anspruch 2 angegebenen Lösung nun - in außerhalb der photodynamischen Diagnose an sich bekannter Weise - eine breitbandige Lichtquelle verwendet, die inkohärentes Licht im Wellenlängenbereich von wenigstens 380 bis 660 nm - bevorzugt von 370 bis 780 nm - abgibt. Diese breitbandige Lichtquelle kann eine andere Spektralverteilung wie herkömmliche, in der Endoskopie verwendete Lichtquellen haben. Wenn die Lichtquelle aber auch zu einer herkömmlichen Untersuchung des - auch - mit photodynamischer Diagnose untersuchten Bereichs verwendet werden soll, ist es bevorzugt, wenn die Lichtquelle eine herkömmliche Hochleistungslichtquelle ist, wie sie in der medizinischen Endoskopie verwendet wird. Die Leistungsaufnahme der Lichtquelle beträgt bevorzugt wenigstens 300 Watt.

Bei der Verwendung von "breitbandigen" Lichtquellen ist in der Vergangenheit das Problem aufgetreten, daß das an dem zu untersuchenden Gewebebereich reflektierte Licht der Lichtquelle das Fluoreszenzlicht überstrahlt. Deshalb ist erfindungsgemäß nicht nur der Reintransmissionsgrad $Ti_l(\lambda)$ der lichtzuführenden Einheit dem Fluoreszenzanregungsspektrum des Photosensibilisators und der Reintransmissionsgrad $Ti_b(\lambda)$ der bildgebenden Einheit dem Fluoreszenzspektrum des Photosensibilisators angepaßt.

Erfindungsgemäß weist bei der im Anspruch 1 angegebenen Lösungsalternative der Reintransmissionsgrad des aus lichtzuführender Einheit und aus bildgebender Einheit bestehenden Gesamtsystems, der sich durch die Mulitiplikation der Reintransmissionsgrade $Ti_l(\lambda)$ und $Ti_b(\lambda)$ ergibt, nur in einem Bereich von maximal 50 nm einen spektralen Transmissionsgrad von mehr als 5% auf und ist ansonsten kleiner als 5%. In diesem Bereich von maximal 50 nm kann der Reintransmissionsgrad des Gesamtsystems Werte von 10% und mehr erreichen.

Bei der im Anspruch 2 angegebenen Alternative weist der spektrale Reintransmissionsgrad $Ti_l(\lambda)$ der lichtzuführenden Einheit zusätzlich zu einem ersten Durchlaßbereich, der dem Fluoreszenzanregungsspektrum des Photosensibilisators bzw. des Gewebes angepaßt ist, einen zweiten Durchlaßbereich auf, dessen Wellenlängen zwischen den Wellenlängen des Fluoreszenzanregungsspektrums und den Wellenlängen des Fluoreszenzspektrums liegt. Der spektrale Reintransmissionsgrad $Ti_b(\lambda)$ der bildgebenden Einheit weist zusätzlich zu einem ersten Durchlaßbereich, der dem Fluoreszenzspektrum des Photosensibilisators bzw. des Gewebes angepaßt ist, einen zweiten Durchlaßbereich auf, der im gleichen Wellenlängenbereich wie der zweite Durchlaßbereich der lichtzuführenden Einheit liegt. Der Reintransmissionsgrad des aus lichtzuführender Einheit und aus bildgebender Einheit bestehenden Gesamtsystems weist dabei nur in dem zweiten Durchlaßbereich einen spektralen Transmissionsgrad von mehr als 5%, der bevorzugt 10% und mehr betragen kann, auf und ist ansonsten in dem obigen Wellenlängenbereich kleiner als 5%.

Durch diese Ausbildung der spektralen Durchlässigkeiten der lichtzuführenden Einheit und der bildgebenden Einheit wird erreicht, daß das Fluoreszenzlicht auf dem durch das Beleuchtungslicht erzeugten Bild beispielsweise der Umgebung eines Tumors klar und kontrastreich wahrgenommen werden kann.

Dabei ist es bevorzugt, wenn der erste und der zweite Durchlaßbereich der bildgebenden Einheit in etwa komplementär zueinander sind. Hierdurch erhält man einen starken Kontrast zwischen dem Fluoreszenzbild und dem beleuchteten Hintergrundbild, der weiter durch eine abwechselnde Darstellung beider Bilder auf einem Monitor gesteigert werden kann.

Weiterhin ist es bevorzugt, wenn die Intensität des Beleuchtungslichts und die Filter so gewählt werden, daß die auf der proximalen Bildebene ankommende Leistung des Lichts mit Wellenlängen aus dem zweiten Bereich 10% (und weniger) bis ca. 100% der kumulierten Lichtleistung im ersten Durchlaßbereich der bildgebenden Einheit ist.

Zur Anpassung an die verschiedenen Photosensibilatoren und/oder unterschiedliche diagnostische Bedingungen oder zur Umstellung der erfindungsgemäßen Vorrichtung auf ein therapeutisches Verfahren ist es weiterhin bevorzugt, wenn die Transmissionseigenschaften der lichtübertragenden und der bildgebenden Einheit mittels eines oder mehrerer optischer Elemente einstellbar sind.

Dabei ist es bevorzugt, wenn die Einstellung derart erfolgt, daß die Intensität des induzierten Fluoreszenzlichtes in der gleichen Größenordnung wie die Gesamtintensität des reflektierenden Anteils des Lichtes des Beleuchtungssystems liegt. Besonders vorteilhafter Weise erfolgt die Einstellung derart, daß die beiden Intensitäten in etwa gleich sind.

In jedem Falle hat die erfindungsgemäße Vorrichtung den Vorteil, daß es zur Durchführung der photodynamischen Diagnose bei gleichzeitiger Beleuchtung des beobachteten Feldes sowie zur vorhergehenden und/oder anschließenden visuellen Beobachtung des untersuchten Bereichs ausreicht, eine einzige Lichtquelle zu verwenden. Darüberhinaus kann mit der erfindungsgemäßen Vorrichtung auch die photodynamische Therapie ohne Änderung der Lichtquelle, d.h. mit einer einzigen Lichtquelle ausgeführt werden. In diesem Falle werden - wie bereits erwähnt - die Transmissionseigenschaften der lichtübertragenden Einheit dem Absorptionsspektrum des Photosensibilisators angepaßt.

Die optischen Elemente, die zur Einstellung der Transmissionseigenschaften der lichtübertragenden und der bildgebenden Einheit verwendet werden, sind bevorzugt Filtersysteme, die in den Beleuchtungs- und den Beobachtungs-Strahlengang einbringbar sind. Dabei wird unter Beleuchtungsstrahlengang der Strahlengang von der Lampe der Lichtquelle zur lichtzuführenden Einheit, durch diese Einheit, und von dieser Einheit zum diagnostizierenden Gewebebereich verstanden. Die optischen Elemente und insbesondere die Filtersysteme können prinzipiell an jeder Stelle dieses Strahlengangs angeordnet sein. Besonders bevorzugt ist jedoch die Anordnung zwischen Beleuchtungssystem und lichtzuführender Einheit, also beispielsweise einem Lichtleiter-Faserbündel. (Ohne Filtersystem wird der Reintransmissionsgrad als 100% angenommen.)

Entsprechend wird unter Beobachtungs-Strahlengang der Strahlengang von dem beleuchteten Gewebebereich zur bildgebenden Einheit und von dieser zur proximalen Bildebene verstanden. (Ohne Filtersystem wird auch hier der Reintransmissionsgrad als 100% angenommen.)

Wenn die erfindungsgemäße Vorrichtung in ein Endoskop integriert ist, kann sich die Bildebene sowohl in dem Endoskop im Bereich des distalen Endes - beispielsweise bei Verwendung eines distal angeordneten Videochips - als auch im Bereich des proximalen Endes befinden. Im letzteren Falle weist die bildgebende Einheit neben einem Objektiv als bildgebende Einheit beispielsweise ein Relaislinsensystem oder ein flexibles Faserbündel als bildübertragende Einheit auf. Bei Verwendung eines Relaislinsen-Systems oder eines Faserbündels als bildübertragende Einheit werden die in dem Beobachtungsstrahlengang eingebrachten Filtersysteme bevorzugt zwischen der "letzten Fläche" des Relaislinsen-Systems bzw. der Austrittsfläche des Faserbündels und der proximalen Bildebene angeordnet.

Bei Integration der erfindungsgemäßen Vorrichtung in ein Operationsmikroskop ist Bestandteil der bildgebenden Einheit das Mikroskop-Linsensystem, dem beispielsweise ein Videoaufnehmer als bilderfassende Einheit nachgeordnet sein kann.

Wie bereits ausgeführt, wird erfindungsgemäß erreicht, daß das von dem zu diagnostizierenden Gewebebereich und dessen Umgebungsbereich zurück reflektierte Beleuchtungslicht das Fluoreszenzlicht nicht überstrahlt. Zur Realisierung dieses erfindungsgemäßen Grundgedankens ist es bevorzugt, wenn das in den Beleuchtungs-Strahlengang und das in den Beobachtungs-Strahlengang jeweils einbringbare Filtersystem nahezu gegenläufige Filtercharakteristiken haben. Im Falle einer gemäß Anspruch 2 ausgebildeten Vorrichtung bezieht sich die gegenläufige Ausbildung der Filtercharakteristik natürlich nur auf die den ersten Durchlaßbereich betreffende Charakteristik, nicht jedoch auf die den zweiten Durchlaßbereich betreffende Charakteristik.

Die Kurven, die die Transmission der beiden gegenläufigen Filter in Abhängigkeit von der Wellenlänge angeben, schneiden sich - bei der Ausgestaltung nach Anspruch 1 - bevorzugt bei einer Transmission der Einzelsysteme, die kleiner als 50 % ist.

Bei einer weiteren Ausgestaltung der Erfindung weist das in den Beleuchtungs-Strahlengang einbringbare Filter mindestens zwei getrennte Filter auf, von denen ein Filter ein thermostabiles Interferenzfilter und das andere Filter ein thermostabiles Wärmeschutzfilter (Neutralfilter) ist.

Im folgenden sollen die Eigenschaften dieser beiden Filter unter der Voraussetzung erläutert werden, daß als Photosensibilisator Delta-Aminolävulinsäure verwendet wird. Bei Verwendung eines anderen Photosensibilisators sind die Filtereigenschaften entsprechend anzupassen:

Bei Verwendung von Delta-Aminolävulinsäure (ALA) ist es bevorzugt, wenn die Transmission des Beleuchtungs-Strahlengangs durch ein Kurzpaß-Filter im Bereich zwischen 380 und 430 nm wenigstens 50 % beträgt. Bei einer besonders bevorzugten Ausführungsform beträgt die Transmission zwischen 370 und 440 nm wenigstens 70 % und bevorzugt 95 %.

Bei einer Wellenlänge von $445 \pm 4$ nm bzw. $447 \pm 2$ nm erreicht die Transmission 50 %. Bei größeren Wellenlängen ist die Transmission sehr viel kleiner und liegt typischerweise unter 1 %.

Bei einer bevorzugten Ausgestaltung der Erfindung liegt die Transmission sowohl im Wellenlängenbereich zwischen 460 und 600 nm als auch zwischen 720 - bevorzugt 660 nm - und 780 nm unter 1 %. In dem Wellenlängenbereich, in dem hauptsächlich Fluoreszenzlicht angeregt wird, also bei Delta-Aminolävulinsäure in dem Wellenlängenbereich zwischen 600 und 720, bevorzugt 660 nm, beträgt die Transmission weniger als 0,1 %.

Durch diese Charakteristik des Filtersystems ergibt sich eine Lichtverteilung des Beleuchtungslichtes, die gewährleistet, daß von dem zu untersuchenden Gewebebereich in dem Wellenlängenbereich, in dem hauptsächlich Fluoreszenzlicht auftritt, praktisch kein "Nicht-Fluoreszenzlicht" zurückgestrahlt wird.

Entsprechend hat das Filter im Beobachtungs-Strahlengang (Langpaßfilter) folgende Charakteristik:

$$T_I(\lambda = 370 - 430 \text{ nm}) < 0,1 \text{ %}$$

$$T_I(\lambda = 453 \pm 2 \text{ nm}) = 50 \text{ %}$$

$$T_i(\lambda = 500 - 1100 \text{ nm}) = 95 \text{ %, bevorzugt } 98-99 \text{ %}$$

Die Toleranz für die Wellenlängen beider Filter, bei der die jeweilige Transmission 50 % beträgt, ist bevorzugt $\pm$ 2 nm. Durch diese Ausbildung der Filter ist gewährleistet, daß die Reintransmission des Gesamtsystems lediglich im Bereich zwischen 430 und 460 nm größer als 5 % ist. Der in diesem Bereich erreichte Maximalwert sollte bevorzugt nicht mehr als 15 % betragen.

Die Verwendung von optischen Elementen und insbesondere von Filtern zur Beeinflussung der Strahlengang-Transmissionscharakteristik hat den Vorteil, daß beispielsweise durch Ausschwenken der Filter eine normale Weißlicht-Beleuchtung und -Beobachtung erfolgen kann, so daß die Untersuchungsperson, also beispielsweise ein Arzt den auch mit Fluoreszenzdiagnose untersuchten Gewebebereich u.a. nach der Farbe beurteilen kann. Die Farbe ist beispielsweise im Bereich der Ophthalmologie ein wesentliches Beurteilungskriterium.

Der bei einer bevorzugten Ausführungsform der Erfindung weiterhin verwendete thermostabile Wärmeschutzfilter (Neutralfilter) kann folgende Charakteristik aufweisen:

$$T_I(\lambda = 370 - 440 \text{ nm}) > 95 \text{ %}$$

$$T_I(\lambda = 440 - 700 \text{ nm}) \approx 90 \text{ %}$$

$$T_I(\lambda = 700 \text{ nm}) = 50 \text{ %}$$

$$T_I(\lambda = 720 - 1100 \text{ nm}) < 1 \text{ %}$$

Die Verwendung eines thermostabilen Wärmeschutzfilters hat den Vorteil, daß der Interferenzfilter während der Diagnose nicht durch Infrarotlicht aufgeheizt wird. Diese Aufheizung könnte gegebenenfalls die Filtercharakteristik verändern und die Empfindlichkeit eines Kameraaufnahmeelements reduzieren sowie die lichtübertragende Einheit aufgrund der hohen Intensität zerstören.

In jedem Falle ist es bevorzugt, wenn die einzelnen Filter nur bei Bedarf in die jeweiligen Strahlengänge eingebracht werden, wobei ihr Entfernen aus dem Strahlengang gegebenenfalls durch ein Überwachungssignal ermöglicht bzw. verhindert wird.

Als Filter können handelsübliche Filter mit der erfindungsgemäß vorgesehenen "nahezu stufenförmigen" Charakteristik verwendet werden, also beispielsweise bekannte Interferenzfilter, deren Trägermaterial Quarz ist.

Als Lichtquellen können ebenfalls bekannte Lichtquellen und insbesondere bereits aus der Endoskopie bekannte Lichtquellen verwendet werden, die breitbandig in dem genannten Wellenlängenbereich Licht emittieren. Eine derartige Lichtquelle, die Licht in ausreichender Intensität emittiert, ist beispielsweise eine Gasentladungslampe und insbesondere eine Xenon-Gasentladungs-Hochdrucklampe. Sollte im Einzelfall die Lichtleistung der Lichtquelle nicht ausreichend sein, kann zusätzlich zu einer "kontinuierlich arbeitenden" Lichtquelle eine "gepulste" Lichtquelle, wie ein Blitzgerät oder auch ein Laser eingesetzt werden. Insbesondere bei der im Anspruch 2 angegebenen alternativen Lösung ist es möglich, das Licht mit einer Wellenlänge λ im ersten Durchlaßbereich mit einem Laser zu erzeugen, der ein gepulster Laser oder ein Dauerstrichlaser sein kann. Das Licht mit Wellenlängen aus dem zweiten Durchlaßbereich kann dann von einer mehr oder weniger breitbandigen "Weißlichtquelle" stammen.

Als lichtzuführende Einheiten können - insbesondere bei endoskopischen Anwendungen - handelsübliche Lichtleiter mit wenigstens einer Faser eingesetzt, die vorteilhafter Weise eine numerische Apertur von mehr als 0,45 besitzen, da dann ein effizienter Lichttransport zu dem zu diagnostizierenden Bereich möglich wird.

Derartige Fasern weisen beispielsweise einen Kern aus Quarz und einen Mantel aus einem thermostabilen Material auf.

Im Falle der Verwendung einer Lichtleitfaser ist es bevorzugt, wenn die Lichtquelle, d.h. also beispielsweise die Gasentladungslampe, einen Brennfleck mit einem Durchmesser von weniger als 2 mm hat, der durch einen elliptischen Reflektor erzeugt wird, der eine numerische Apertur für den Lichtaustritt von mehr als 0,45 aufweist. In diesem Falle erhält man eine hocheffiziente Ankopplung zwischen Beleuchtungssystem und lichtzuführender Einheit, also dem Faser-Lichtleiter.

Ferner kann auch eine Gasentladungslampe verwendet werden, deren Brennfleck auf einen Durchmesser von weniger als 2 mm mittels eines parabolischen Reflektors und einer Fokussiereinheit fokussiert wird. Die Fokussiereinheit ist in diesem Falle bevorzugt ein Linsensystem, das wenigstens ein Element mit einer asphärischen Fläche aufweist.

Bei einer weiteren bevorzugten Ausgestaltung der Erfindung ist das Beleuchtungssystem, also die Lichtquelle und die der Lichtquelle vorgeschalteten optischen Elemente, wie Filter etc., so ausgebildet, daß die Anregungswellenlängen entsprechend dem jeweils eingesetzten Photosensibilisator und dem jeweils zu diagnostizierenden Gewebe durchstimmbar sind. Diese Durchstimmung kann entweder durch eine entsprechende Beeinflussung der Lichtquelle oder mittels vorgeschalteter Filter, wie z.B. Verlaufsfilter oder Prismen erfolgen.

Damit ist es u.a. möglich, gezielt unterschiedliche Gewebebereiche zur Fluoreszenz anzuregen.

Die erfindungsgemäße Vorrichtung ermöglicht sowohl eine visuelle Beobachtung der Fluoreszenz als auch die Aufnahme des Fluoreszenzbildes mit einem Bildaufnehmer, wie einer Videokamera oder dgl.

Diese Videokamera bzw. -einheit wird in der Bildebene der bildgebenden Einheit angeordnet. Im Falle einer distalen Anordnung der Videoeinheit wird diese in der Bildebene des Objektivs des Endoskops angeordnet. Im Falle einer proximalen Anordnung der Videoeinheit wird diese in der Bildebene der Bildweiterleitereinheit, also des Relaislinsensystems oder des Faserbündels angeordnet. Alternativ kann die Videoeinheit so ausgebildet sein, daß sie das Okularbild aufnimmt. Bei Verwendung eines Mikroskops als bildgebende Einheit wird die Videoeinheit so angeordnet, daß sie das Okularbild des Operationsmikroskops aufnimmt.

Die Videoeinheit kann insbesondere (wenigstens) einen CCD-Aufnehmer aufweisen. In diesem Falle ist es möglich, daß die Gasentladungslampe eine periodisch arbeitende Blitzentladungslampe ist, die von einer Steuer- und Auswerteeinheit so angesteuert wird, daß die Blitzbelichtung ausschließlich in die Lichtintegrationsphase des oder der CCD-Aufnehmer fällt. Damit wird eine hocheffektive Beleuchtung des zu untersuchenden Bereichs erzielt, wobei die Beaufschlagung des Beleuchtungssystems und der Umgebung mit Lichtenergie verringert wird. Damit wird auch die Wärmebzw. Temperaturbelastung der Bauteile verringert.

Soll gleichzeitig eine visuelle Beobachtung des zu untersuchenden Bereichs erfolgen oder die Lichtleistung gesteigert werden, ist es von Vorteil, wenn zusätzlich eine kontinuierlich arbeitende Lichtquelle vorgesehen ist.

Zur Steuerung des Videosignals ist es weiterhin bevorzugt, wenn die Videoeinheit eine variable Belichtungseinstellung aufweist; damit können Überstrahlungen im Videobild verhindert werden und man erhält immer ein kontrastreiches Bild, das die Fluoreszenzstrahlung gut erkennen läßt.

Eine erfindungsgemäße Vorrichtung, die die vorstehend beschriebenen Merkmale aufweist, ermöglicht die visuelle Beobachtung des Fluoreszenzbildes mittels des bloßen Auges oder einer Videoeinheit. Ein besonderer Vorteil der erfindungsgemäßen Vorrichtung ist jedoch, daß das von ihr erzeugte Bild eine weitgehend automatisierte Auswertung erlaubt:

Hierzu ist das Ausgangssignal der Videoeinheit an ein Bildverarbeitungssystem angelegt. Dieses Bildverarbeitungssystem kann eine Reihe von Manipulationen in dem von der Videoeinheit gelieferten Bild ausführen:

Die Farbbilder können beispielsweise über die RGB-Eingangskanäle erfaßt und in den HSI-Farbraum transformiert werden:

(H = Hue (Farbton)
(S = Saturation)
(I = Intensität) .

Im HSI-Raum wird die beispielsweise durch Tumore hervorgerufenen "Fluoreszenzstrahlung" durch die erfolgte HSI-Trennung hervorgehoben.

Weiterhin ist es möglich, daß das Bildverarbeitungssystem elektronisch einen Farbtonbereich im dargestellten Farbbild ausblendet, um den Kontrast zwischen unterschiedlichen Bereichen anzuheben. Wenn das Bildverarbeitungssystem eine RGB-Erfassung ausführt, kann beispielsweise zudem der Blau- und/oder Grünkanal an- und abgeschaltet werden. Diese Vorgehensweise hat den Vorteil, daß bei Abschaltung des Blau-und/oder Grünkanals das Fluoreszenzbild besonders deutlich hervortritt.

Diese Hervorhebung wird dadurch verstärkt, daß das Bildverarbeitungssystem den ausgeblendeten Farbkanal shutterartig in das beispielsweise auf einem Monitor dargestellte Farbbild einblendet. Hierdurch ergibt sich für den Betrachter eine besonders "ins Auge stechende" Darstellung, die insbesondere die Erkennung von Tumoren vereinfacht.

Ein Untersuchungsvorgang kann dabei etwa wie folgt ablaufen:

Zunächst wird der zu untersuchende Gewebebereich visuell untersucht. Damit ist gemeint, daß ein Arzt den mit "weißem" Licht beleuchteten Bereich mit dem Okular eines Endoskops bzw. eines Mikroskops oder auf einem Monitor betrachtet. Zur Umschaltung auf photodynamische Diagnose wird beispielsweise mittels eines Fußschalters oder eines Schalters an der Videokamera der Kurzpaßfilter und gegebenenfalls der thermostabile Wärmeschutzfilter in den Beleuchtungsstrahlengang eingeschwenkt. Gleichzeitig wird der Grünkanal und/oder der Blaukanal periodisch abgeschaltet. Damit sieht der Arzt auf dem Monitor einmal nur das Fluoreszenzbild (oder das "normale" Bild) und dann die Überlagerung des Fluoreszenzbildes mit dem "normalen" Bild, das durch Licht aus dem kleinen Bereich, in dem die Transmission des Gesamtsystems ungleich 0 ist -also entweder dem Überlappungsbereich oder dem zweiten Durchlaßbereich -, erzeugt wird. Umgekehrt kann auch das Fluoreszenzbild periodisch dem Hintergrundbild (Blaukanal) überlagert werden.

Weiterhin kann das Bildverarbeitungssystem zur Bestimmung eventueller Tumore den Fluoreszenz-Kontrastwert an den einzelnen Stellen des Bildes für die maximale Fluoreszenzwellenlänge berechnen. Im Falle der Verwendung von Delta-Aminolävulinsäure (ALA) als Photosensibilisator kann das Kontrastverhältnis für die Wellenlänge 630 nm zur Intensität in dem Bereich von maximal 50 nm berechnet, in dem das Gesamtsystem einen spektralen Transmissionsgrad von mehr als 5% aufweist:

Durch einen Vergleich der mit und ohne Fluoreszenzanregung aufgenommenen Bilder - im einfachsten Falle eine Subtraktion der Bilder - kann die Bildverarbeitungseinheit die Intensität der Fluoreszenzstrahlung bestimmen und das Kontrastverhältnis berechnen, so daß eine genaue Lokalisierung eventueller Tumore möglich ist.

Die erfindungsgemäße Vorrichtung kann für die verschiedensten medizinischen Untersuchungen eingesetzt werden:

Neben dem besonders bevorzugten Einsatz in endoskopischen Anwendungen kann die erfindungsgemäße Vorrichtung auch in Verbindung mit einem Operationsmikroskop beispielweise in der Neurochirurgie, der Kolposkopie oder der Ophthalmologie eingesetzt werden.

In jedem Falle erhält man jedoch eine Vorrichtung, die insbesondere folgende Vorteile aufgrund der Mitbeobachtung von an dem Gewebe reflektierten Beleuchtungslicht zugleich oder alternierend mit dem Fluoreszenzlicht aufweist:

- Es ist eine Orientierung auch über fluoreszenznegativem Gewebe möglich.

- Die erfindungsgemäß realisierte Orientierung hat gegenüber einer reinen Weißlicht-Beleuchtung insbesondere bei der Verwendung von ALA als Photosensibilisator den Vorteil, daß eine starke Betonung der Gefäßstrukturen erfolgt und sich klare Sichtverhältnisse auch bei diffusen Einblutungen in die Spülflüssigkeit ergeben.

- Die Schwellwertfunktion des blauen Lichts unterdrückt die unspezifische Rotfluoreszenz des Normalgewebes.

- Vor allem aber ist die Erkennung echt fluoreszenzpositiver Areale durch Farbkontrast und nicht durch Intensitätskontrast - wie dies bei einer vollständigen Blockung des Beleuchtungslichtes der Fall wäre - möglich. Dies vereinfacht insbesondere die Bildverarbeitung !

- Der erfindungsgemäß erhaltene Farbkontrast ist - im Gegensatz zum Intensitätskontrast - unabhängig vom Beobachtungsabstand und vom Beobachtungswinkel. Damit wird die Irrtumswahrscheinlichkeit wesentlich reduziert. Darüberhinaus erübrigen sich aufwendige Bildverarbeitungsverfahren, so daß im Falle einer automatisierten Erkennung einfache Bildverarbeitungsverfahren zur Anwendung kommen können.

## Kurze Beschreibung der Zeichnung

Die Erfindung wird nachstehend anhand eines Ausführungsbeispiels unter Bezugnahme auf die Zeichnung näher beschrieben, in der zeigen:

Fig. 1 schematisch eine erfindungsgemäße Vorrichtung für endoskopische Anwendungen,

Fig. 2a die Filtercharakteristik der in den einzelnen Strahlengängen eingesetzten Filter,

Fig. 2b die "Gesamttransmission" eines ersten Ausführungsbeispiels, und

Fig. 3a und b entsprechende Darstellungen für ein zweites Ausführungsbeispiel.

## Darstellung von Ausführungsbeispielen

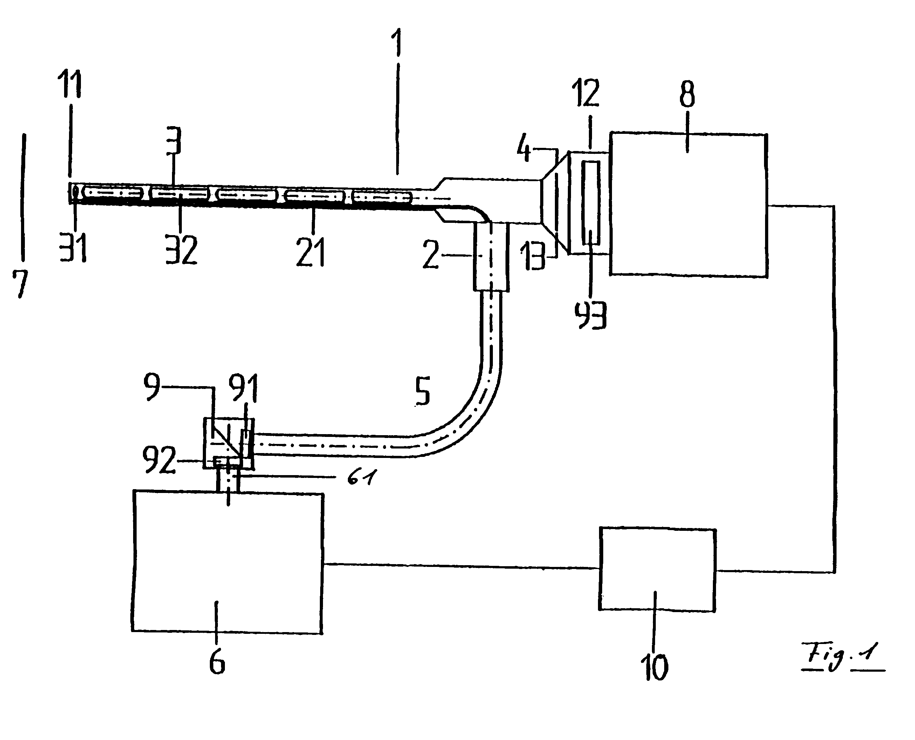

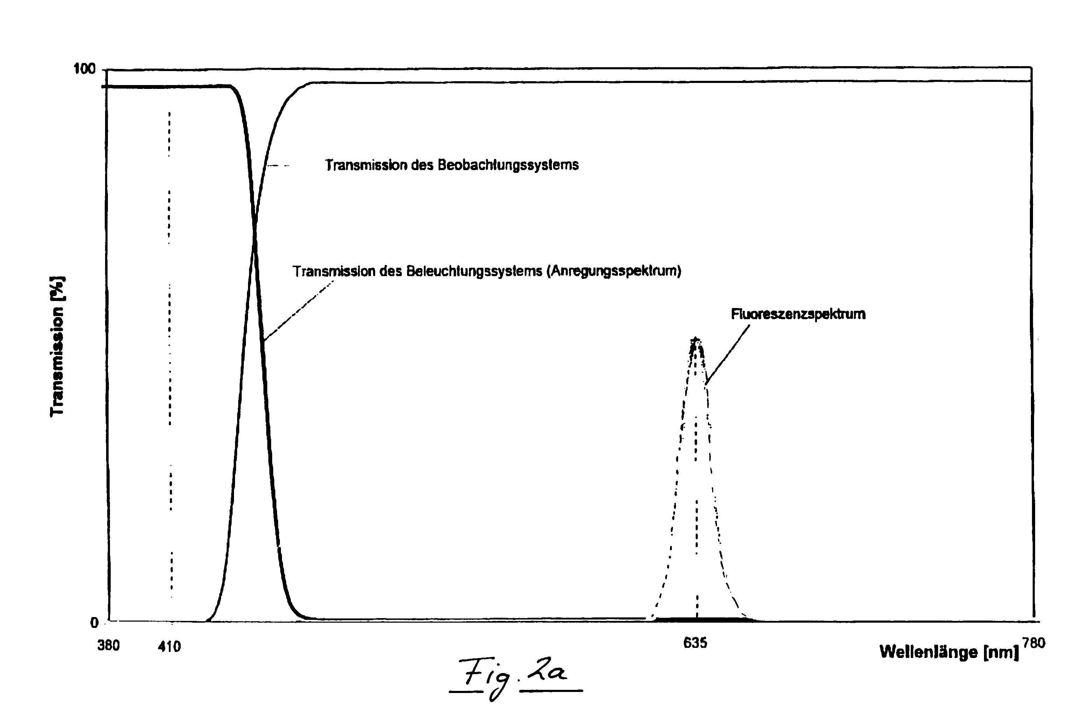

In Fig. 1 ist schematisch der Aufbau einer erfindungsgemäßen Vorrichtung für endoskopische Anwendungen dargestellt. Das Bezugszeichen 1 bezeichnet ein Endoskop, das in bekannter Weise einen Lichtleiteranschluß 2, einen stabförmigen Teil 3, der in einen (nicht dargestellten) menschlichen Körper einführbar ist, und ein Okular 4 aufweist.

Der Lichtleiteranschluß 2 ist über ein Lichtleitkabel 5 mit einer Lichtquelle 6 verbundenen, die beispielsweise eine Xenon-Entladungslampe aufweisen kann. Ein z.B. aus einem Faserbündel bestehender Lichtleiter 21 in dem Endoskop 1 leitet das in den Lichtleiteranschluß 2 eingekoppelte Licht der Lichtquelle 6 zum distalen Ende 11 des Endoskops 1. Das aus dem distalen Ende 11 austretende Licht beleuchtet den zu untersuchenden Gewebebereich 7.

Das von dem Gewebebereich 7 kommende Licht tritt in ein nur schematisch dargestelltes Objektiv 31 des Endoskops 1 ein. Das Bild des Objektivs 31 wird durch einen Bildweiterleiter 32 zum proximalen Ende 12 des Endoskops 1 geleitet.

Bei dem gezeigten Ausführungsbeispiel weist der Bildweiterleiter 32 eine Mehrzahl von Relaislinsensystem auf, von denen jedes eine 1:1-Abbildung ausführt und aus sog. Stablinsen-Systemen besteht. Alternativ kann der Bildweiterleiter 32 ein Faser-Abbildungssystem aufweisen Das in der proximalen Bildebene 13 erzeugte Bild des Gewebebereichs 7 kann durch das Okular 4 mit dem Auge betrachtet werden. Alternativ oder über einen Strahlteiler zusätzlich zur Betrachtung mit dem Auge kann das Bild mit einer Videokamera 8 aufgenommen werden. In Fig. 1 ist die Alternative dargestellt, daß die Videokamera 8 direkt am Okular 4 angebracht ist.

Soweit wie vorstehend beschrieben ist der Aufbau beispielsweise durch mit einer Videokamera versehene Endoskope der Karl Storz GmbH & Co., Tuttlingen, Deutschland bekannt. Zum Detailaufbau wird deshalb auf die bekannten Endoskope dieses Herstellers Bezug genommen.

Zur Durchführung sogenannter photodynamischer Diagnosen ist die in Fig. 1 dargestellte Vorrichtung so weitergebildet, daß in den Beleuchtungsstrahlengang und in den Beobachtungsstrahlengang Filtersysteme eingebracht werden können.

Hierzu ist bei dem in Fig. 1 dargestellten Ausführungsbeispiel an dem Licht-Ausgangsanschluß 61 der Lichtquelle 6 ein Filtersystem 9 angebracht, an dem wiederum das Lichtleitkabel 5 angeflanscht ist. Das Filtersystem 9 weist einen thermostabilen Interferenzfilter 91 und einen thermostabilen Wärmeschutzfilter 92 auf, der im wesentlichen die Wärmebeaufschlagung des Interferenzfilters 91 reduzieren soll. Auch vor der Videokamera 8 oder dem Auge, mit dem das Bild visuell ausgewertet wird, ist ein Filter 93 angebracht.

Die Belichtungseinstellung der Videokamera 8 und die Lichtabgabe der Lichtquelle 6 werden von einer Steuer- und Auswerteeinheit 10 gesteuert. Beispielsweise kann die Steuer- und Auswerteeinheit 10 eine Blitz-Lichtquelle mit der Lichtintegrationsphase eines CCD-Chips in der Videokamera 8 synchronisieren. Ferner kann die Steuer- und Auswerteeinheit 10 die von der Lichtquelle 6 abgegebene Lichtleistung und/oder die Belichtungseinstellung der Videokamera regeln.

An der Steuer- und Auswerteeinheit 10 liegt ferner das Ausgangssignal der Videokamera 8 an. Die Auswerteeinheit kann insbesondere ein Bildverarbeitungssystem aufweisen, das das Ausgangssignal der Videokamera in der einleitend beschriebenen Weise weiterverarbeitet und das bildverarbeitete Ausgangssignal auf einem Monitor darstellt. Selbstverständlich kann das unmittelbar von der Videokamera abgegebene und/ oder das bildverarbeitete Ausgangssignal auch z.B. mittels eines Videorecorders gespeichert werden und/oder in einer Bilddatenbank abgelegt oder in sonstiger Weise mittels elektronischer Datenverarbeitung weiterverarbeitet werden.

Bei Verwendung eines Photosensibilisators geht von dem Gewebebereich 7 sowohl reflektiertes Beleuchtungslicht als auch Fluoreszenzlicht aus, das durch die von dem Photosensibilisator lichtinduzierte Reaktion in biologischen Systemen hervorgerufen wird. Um den verglichen mit dem reflektierten Licht geringen Anteil an Fluoreszenzlicht nachweisen und insbesondere bei einer nach folgenden Bildverarbeitung sicher von dem "Nicht-Fluoreszenzlicht" trennen zu können, ist eine geeignet gewählte Transmissionscharakteristik des Beleuchtungs- und des Beobachtungsstrahlengangs erforderlich. Zur Einstellung der Transmissionscharakteristik während der photodynamischen Diagnose dienen die in den Strahlengang ein bringbaren Filter 91 und 93. Da die Filter beispielsweise durch Ausschwenken aus den Strahlengängen wieder entfernt werden können, ist auch eine normale Beobachtung des Gewebebereichs 7 möglich, ohne daß es beispielsweise zu einer Farbverfälschung kommen würde.

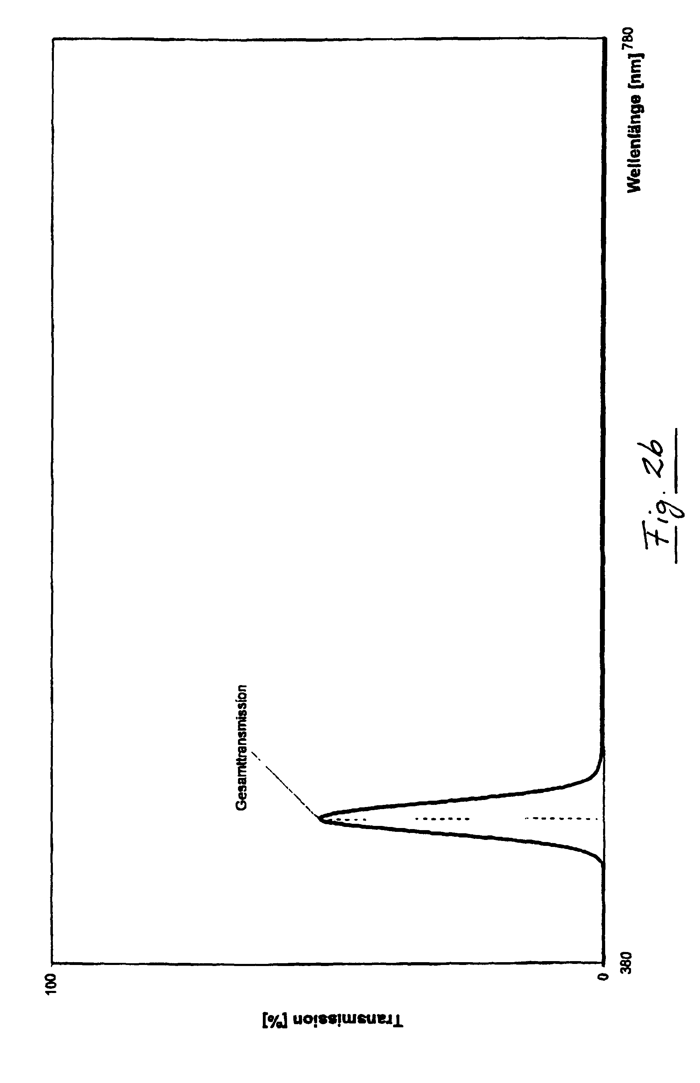

Nachfolgend soll unter Bezugnahme auf Fig. 2 die Charakteristik der Filter 91 und 93 für die erste Ausführungsform der Erfindung sowie für den Fall erläutert werden, daß Delta-Aminolävulinsäure als Photosensibilisator verwendet wird. Bei Verwendung anderer Photosensibilisatoren ist die Filtercharakteristik entsprechend anzupassen.

In Fig. 2a stellt die dick ausgezogene Kurve die Transmission (in Prozent) des Filters 91 als Funktion der Wellenlänge (in nm), also den sog. Reintransmissionsgrad $Ti(\lambda)$ dar, während die dünn ausgezogene Kurve den Reintransmissionsgrad $Ti(\lambda)$ für das Filter 93 wiedergibt. Ferner ist in Fig. 2a das Fluoreszenzspektrum eingetragen.

Fig. 2a ist zu entnehmen, daß die Reintransmission des Filters 91 bei Wellenlängen, die kleiner als etwa 440 nm sind, größer als 90% ist. Bei Wellenlängen, die größer als etwa 460 nm sind, ist die Transmission kleiner als 1%. Etwa bei 455 nm ist die Transmission 50 %. Ferner ist bei dem Ausführungsbeispiel für ein Interferenzfilter 91, das in Fig. 2a dargestellt ist, die Transmission im Bereich zwischen 600 und 660 nm - bevorzugt jedoch 720 bis 780 nm -, in dem Fluoreszenzlicht verstärkt auftritt, besonders niedrig und ist insbesondere kleiner als 0,1 %, bevorzugt 0,01 %. Das Filter 91 ist also ein Kurzpaßfilter.

Das Filter 93 hat eine nahezu gegenläufige Charakteristik:

Die Transmission $T_l$ ist bei Wellenlängen zwischen 370 und 430 nm kleiner als 0,1 %, bevorzugt sogar um wenigstens eine Größenordung kleiner als 0,1 %. Bei einer Wellenlänge von 445 nm ist die Transmission 50 %. Bei Wellenlängen zwischen 500 und 1100 nm erreicht die Transmission nahezu 99 % oder mehr.

Die Transmissionskurven der Filter 91 und 93 schneiden sich bei dem gezeigten Ausführungsbeispiel ca. bei Durchlaßwerten von mehr als 60%. Es ist jedoch auch möglich - und insbesondere bei schwacher Fluoreszenz bevorzugt, daß sich die Kurven bei Durchlaßwerten von 25 bis 30% schneiden.

Fig. 2b zeigt die Transmissionscharakteristik des Gesamtsystems, die man durch Multiplikation der Kurven der einzelnen Filter 91 und 93 erhält. Wie man sieht, liegt die Transmission nur im Bereich zwischen 440 und 460 nm über 1% und erreicht einen Maximalwert von etwa 50%.

Bei der vorstehend genannten Alternative, bei der sich die Kurven bei Durchlaßwerten von 25 bis 30 % schneiden, beträgt der Maximalwert ca. 12,5 %. Dies bedeutet, daß nur ein kleiner Teil des Beleuchtungslichtes, das an dem Gewebebereich 7 reflektiert wird, die proximale Bildebene 13 "erreicht". Das Fluoreszenzlicht, das typischerweise bei Wellenlängen $\lambda$ zwischen 500 und 780nm, meist zwischen 600 und 660 nm emittiert wird, erreicht die Bildebene 13 ungehindert, da es nur das Langpaßfilter 93, nicht jedoch das Kurzpaßfilter 91 passieren muß.

Damit ist zwar noch eine visuelle Beobachtung des Gewebebereichs 7 möglich, der Fluoreszenzanteil des nachgewiesenen Lichtes kann aber sicher von dem reflektierten, kürzerwelligen Licht getrennt werden.

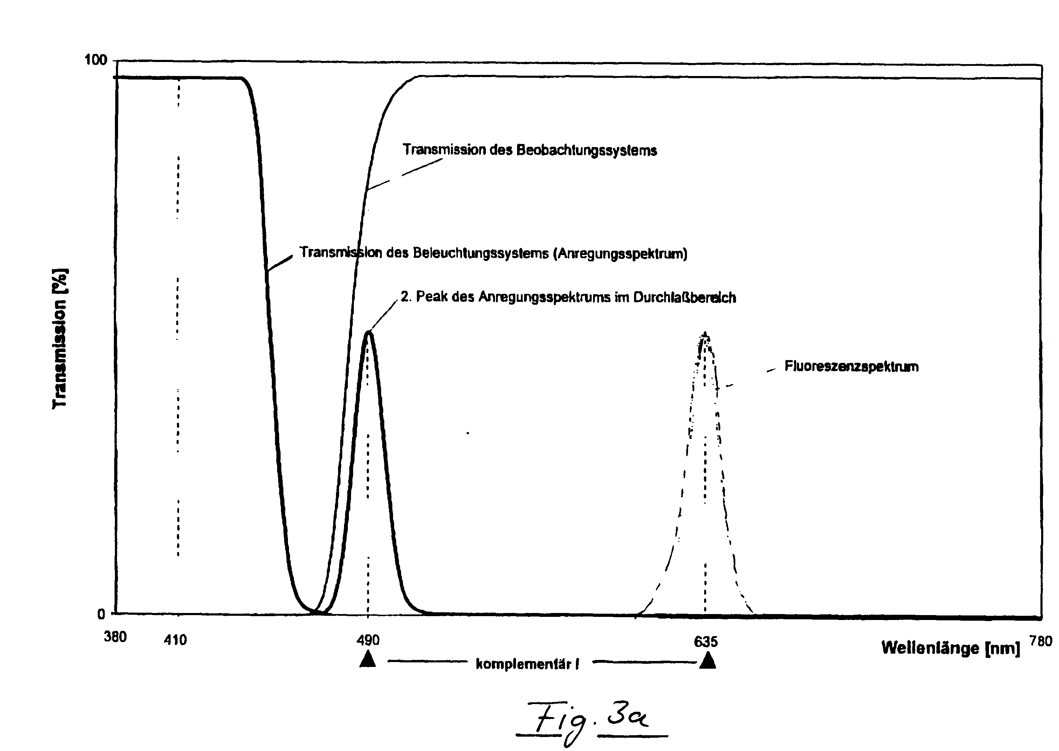

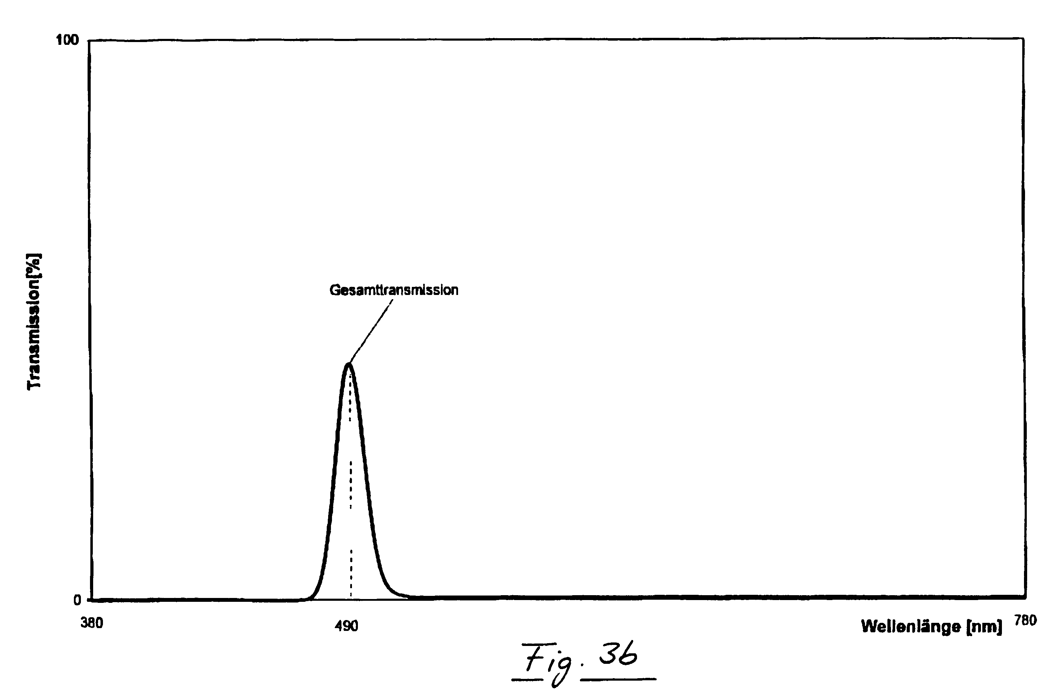

Die Fig. 3a und 3b zeigen die entsprechenden Kurven für die zweite Ausführungsform der Erfindung, bei der sowohl das im Beleuchtungsstrahlengang eingebrachte Filter 91 als auch das im Beobachtungsstrahlengang vorgesehene Filter 93 einen zweiten Durchlaßbereich bei ca. 490 nm ± 10 nm aufweisen.

Die vorstehenden Wellenlängenangaben beziehen sich auf die Verwendung von Delta-Aminolävulinsäure als Photosensibilisator. Bei der Verwendung von anderer Photosensibilisatoren oder bei Ausnutzung der Eigenfluoreszenz sind die Wellenlängen, bei denen die einzelnen Filter ihre Paß-Charakteristik ändern, entsprechend anzupassen. Unverändert bleibt jedoch im wesentlichen die Eigenschaft, daß sich die beiden komplementären Filterkurven bei einem Reintransmissionsgrad von ca. 50 % oder weniger als 50% schneiden, oder daß ein zweiter Durchlaßbereich vorhanden ist. Ebenfalls unverändert bleibt, daß das Gesamtsystem nur in einem schmalen Bereich, der typischerweise 50 nm, gegebenenfalls aber mehr oder weniger, beträgt, deutlich von Null verschieden und insbesondere größer als 1% ist.

Soll die erfindungsgemäße Vorrichtung auch für die Therapie verwendet werden, so muß das Filtersystem 9 ein weiteres Filter aufweisen, das keine Kurzpaß-Charakteristik, sondern eine Mittelpaß- bis Langpaßcharakteristik aufweist.

Die vorstehende Beschreibung von Ausführungsbeispielen, die sich auf die Verwendung von Verwendung von Delta-Aminolävulinsäure als Photosensibilisator bezieht, beschränkt den den Ansprüchen und der Beschreibung entnehmbaren allgemeinen Erfindungsgedanken nicht.

**Patentansprüche**

1. Vorrichtung zur Diagnose mittels einer durch einen Photosensibilisator lichtinduzierten oder durch Eigenfluoreszenz hervorgerufenen Reaktion in biologischem Gewebe "in vivo", mit

   - einem Beleuchtungssystem, das mindestens eine Lichtquelle mit einem Lampensystem (6) aufweist, das inkohärentes Licht im Wellenlängenbereich von wenigstens 380 bis 680 nm erzeugt,
   - einer lichtzuführenden Einheit (2,5,21), die das Licht des Beleuchtungssystems auf den zu diagnostizierenden und/oder zu therapierenden Gewebebereich (7) richtet, und

- einer bildgebenden, bilderfassenden sowie bildübertragenden Einheit (31,32,4,8), die das von dem Gewebebereich (7) kommende Licht in eine proximale Bildebene (13) abbildet,

wobei der spektrale Reintransmissionsgrad Tl($\lambda$) der lichtzuführenden Einheit dem Fluoreszenzanregungsspektrum des Photosensibilisators bzw. des Gewebes und der spektrale Reintransmissionsgrad Tb($\lambda$) der bildgebenden Einheit dem Fluoreszenzspektrum des Photosensibilisators bzw. des Gewebes angepaßt ist, und wobei der Reintransmissionsgrad des aus lichtzuführender Einheit und aus bildgebender Einheit bestehenden Gesamtsystems nur in einem Wellenlängenbereich von maximal 50 nm einen spektralen Transmissionsgrad von mehr als 5% aufweist und ansonsten in dem oben genannten Wellenlängenbereich kleiner als 5% ist.

2. Vorrichtung zur Diagnose mittels einer durch einen Photosensibilisator lichtinduzierten oder durch Eigenfluoreszenz hervorgerufenen Reaktion in biologischem Gewebe "in vivo", mit

- einem Beleuchtungssystem, das mindestens eine Lichtquelle aufweist,
- einer lichtzuführenden Einheit (2,5,21), die das Licht des Beleuchtungssystems auf den zu diagnostizierenden und/oder zu therapierenden Gewebebereich (7) richtet, und
- einer bildgebenden, bilderfassenden sowie bildübertragenden Einheit (31,32,4,8), die das von dem Gewebebereich (7) kommende Licht in eine proximale Bildebene (13) abbildet,

wobei der spektrale Reintransmissionsgrad Ti$_l$($\lambda$) der lichtzuführenden Einheit einen ersten Durchlaßbereich, der dem Fluoreszenzanregungsspektrum des Photosensibilisators bzw. des Gewebes angepaßt ist, und einen zweiten Durchlaßbereich aufweist, dessen Wellenlängen zwischen den Wellenlängen des Fluoreszenzanregungsspektrums und den Wellenlängen des Fluoreszenzspektrums liegt, und der spektrale Reintransmissionsgrad Tb($\lambda$) der bildgebenden Einheit einen ersten Durchlaßbereich, der dem Fluoreszenzspektrum des Photosensibilisators bzw. des Gewebes angepaßt ist, und einen zweiten Durchlaßbereich aufweist, der im gleichen Wellenlängenbereich wie der zweite Durchlaßbereich der lichtzuführenden Einheit liegt, und wobei der Reintransmissionsgrad des aus lichtzuführender Einheit und aus bildgebender Einheit bestehenden Gesamtsystems nur in dem zweiten Durchlaßbereich einen spektralen Transmissionsgrad von mehr als 5% aufweist und ansonsten in dem obigen Wellenlängenbereich kleiner als 5% ist.

3. Vorrichtung nach Anspruch 2, dadurch **gekennzeichnet**, daß der erste und der zweite Durchlaßbereich der bildgebenden Einheit in etwa komplementär zueinander sind.

4. Vorrichtung nach einem der Ansprüche 1 bis 3, dadurch **gekennzeichnet**, daß das Beleuchtungssystem mehrere Lichtquellen aufweist, von denen eine eine breitbandige Lichtquelle mit einem Lampensystem ist und eine weitere wenigstens ein Lasersystem aufweist.

5. Vorrichtung nach Anspruch 4, dadurch **gekennzeichnet,** daß alle Lichtquellen des Beleuchtungssystems in die lichtzuführende Einheit eingekoppelt werden.

6. Vorrichtung nach einem der Ansprüche 1 bis 5, dadurch **gekennzeichnet,** daß die Transmissionseigenschaften der lichtübertragenden und der bildgebenden Einheit mittels eines oder mehrerer optischer Elemente einstellbar sind.

7. Vorrichtung nach Anspruch 6, dadurch **gekennzeichnet,** daß die Transmissionseigenschaften derart eingestellt sind, daß die Gesamtintensität des induzierten Fluoreszenzlichtes in der gleichen Größenordnung wie die Gesamtintensität des direkt an dem Gewebebereich reflektierten Anteils des Lichts des Beleuchtungssystems liegt.

8. Vorrichtung nach Anspruch 6 oder 7, dadurch **gekennzeichnet**, daß die optischen Elemente Filtersysteme (9,91,92,93) sind, die in den Beleuchtungs- bzw. den Beobachtungs-Strahlengang einbringbar sind.

9. Vorrichtung nach Anspruch 8, dadurch **gekennzeichnet**, daß das in den Beleuchtungs-Strahengang einbringbare Filtersystem und das in den Beobachtungs-Strahlengang einbringbare Filtersystem eine annähernd komplementäre Filtercharakteristik haben.

10. Vorrichtung nach Anspruch 9 in Verbindung mit Anspruch 1, dadurch **gekennzeichnet,** daß sich die beiden komplementären Filterkurven bei einem Reintransmissionsgrad der Einzelsysteme von weniger als 50% schneiden.

11. Vorrichtung nach einem der Ansprüche 8 bis 10, dadurch **gekennzeichnet,** daß das Filtersystem im Beleuchtungsstrahlengang mindestens zwei in den Strahlengang einbringbare Filter aufweist, von denen ein Filter ein thermostabiles Interferenzfilter (91) und das andere Filter ein thermostabiles Wärmeschutzfilter (92) ist.

**12.** Vorrichtung nach Anspruch 11, dadurch **gekennzeichnet**, daß bei Verwendung von Delta-Amino-ävulinsäure als Photosensibilisator das thermostabile Interferenzfilter (91) folgende Transmissionseigenschaften im sichtbaren Bereich aufweist:

$$TI(\lambda = 380 - 430 \text{ nm}) > 50\,\%$$

$$TI(\lambda = 610 - 650 \text{ nm}) < 1\,\%$$

**13.** Vorrichtung nach Anspruch 12, dadurch **gekennzeichnet,** daß der Blockungsfaktor des thermostabilen Interferenzfilters (91) bei einer Wellenlänge $\lambda$ von 630 nm bezogen auf das Transmissionsmaximum im Durchlaßbereich bei einer Wellenlänge von ca. 407 nm größer als 1000 ist.

**14.** Vorrichtung nach Anspruch 12 oder 13 in Verbindung mit Anspruch 1, dadurch **gekennzeichnet,** daß das thermostabile Interferenzfilter (Kurzpaßfilter 91) folgende Charakteristik aufweist:

$$TI(\lambda = 370 - 440 \text{ nm}) > 95\,\%$$

$$TI(\lambda = 447 \text{ nm}) = 50\,\%$$

$$TI(\lambda = 460 - 600 \text{ nm}) < 1\,\%$$

$$TI(\lambda = 600 - 660 \text{ nm}) < 0{,}1\,\%$$

$$TI(\lambda = 660 - 680 \text{ nm}) < 1\,\%$$

**15.** Vorrichtung nach Anspruch 12 oder 13 in Verbindung mit Anspruch 2, dadurch **gekennzeichnet,** daß das thermostabile Interferenzfilter (Kurzpaßfilter 91) folgende Charakteristik aufweist:

$$TI(\lambda = 370 - 440 \text{ nm}) > 95\,\%$$

$$TI(\lambda = 447 \text{ nm}) = 50\,\%$$

$$TI(\lambda = 460 - 480 \text{ nm}) < 1\,\%$$

$$TI(\lambda = 490 \text{ nm}) > 10\%$$

$$TI(\lambda = 500 - 600 \text{ nm}) < 1\,\%$$

$$TI(\lambda = 600 - 660 \text{ nm}) < 0{,}1\,\%$$

$$TI(\lambda = 660 - 680 \text{ nm}) < 1\,\%$$

**16.** Vorrichtung nach Anspruch 14 oder 15, dadurch **gekennzeichnet,** daß die Toleranz für die Wellenlänge, bei der die Transmission 50% beträgt, $\pm 2$ nm ist.

**17.** Vorrichtung nach einem der Ansprüche 12 bis 16, dadurch **gekennzeichnet,** daß das thermostabile Wärmeschutzfilter (92) folgende Charakteristik aufweist:

$$T_I(\lambda = 370 - 440 \text{ nm}) > 95\,\%$$

$$T_I(\lambda = 440 - 700 \text{ nm}) \approx 90\,\%$$

$$T_I(\lambda = 700 \text{ nm}) = 50\,\%$$

$$T_I(\lambda = 720 - 1100 \text{ nm}) < 1\,\%$$

**18.** Vorrichtung nach einem der Ansprüche 9 bis 17, dadurch **gekennzeichnet**, daß das thermostabile Interferenzfilter (91) nur für die Durchführung des Diagnosevorgangs in den Beleuchtungs-Strahlengang eingebracht wird.

**19.** Vorrichtung nach einem der Ansprüche 1 oder 3 bis 18, dadurch **gekennzeichnet,** daß die Reintransmission des Gesamtsystems lediglich im Bereich zwischen 430 und 460 nm größer als 5% ist, und in diesem Bereich einen Maximalwert von nicht mehr als ca. 50% bevorzugt ca. 15%-35% erreicht.

**20.** Vorrichtung nach einem der Ansprüche 8 bis 19, dadurch **gekennzeichnet,** daß das Entfernen der Filter (91, 92, 93) aus den Strahlengängen nur möglich ist, wenn ein Überwachungssignal dies freigibt.

**21.** Vorrichtung nach einem der Ansprüche 8 bis 20, dadurch **gekennzeichnet,** daß das Trägermaterial der Filter des Filtersystems Quarz oder ein wärmebeständiges Glasmaterial ist.

**22.** Vorrichtung nach einem der Ansprüche 1 bis 21,

dadurch **gekennzeichnet,** daß die Lichtquelle (6) wenigstens eine Gasentladungslampe aufweist.

23. Vorrichtung nach Anspruch 22,
dadurch **gekennzeichnet,** daß die Gasentladungslampe eine Xenon-Gasentladungslampe ist.

24. Vorrichtung nach einem der Ansprüche 1 bis 23,
dadurch **gekennzeichnet**, daß die lichtzuführende Einheit einen Lichtleiter (21) mit wenigstens einer Faser aufweist, dessen numerische Apertur größer als 0,45 ist.

25. Vorrichtung nach Anspruch 24,
dadurch **gekennzeichnet,** daß das Kernmaterial der Faser aus Quarz und der Mantel aus einem thermostabilen Material besteht.

26. Vorrichtung nach Anspruch 24 oder 25,
dadurch **gekennzeichnet,** daß die Gasentladungslampe des Beleuchtungssystems einen Brennfleck mit einem Durchmesser von weniger als 2mm und einen elliptischen Reflektor aufweist, der eine numerische Apertur für den Lichtaustritt von mehr als 0,45 aufweist.

27. Vorrichtung nach Anspruch 24 oder 25,
dadurch **gekennzeichnet,** daß die Gasentladungslampe des Beleuchtungssystems einen Brennfleck mit einem Durchmesser von weniger als 2mm und einen parabolischen Reflektor sowie als Fokussiereinheit ein Linsensystem mit mindestens einer asphärischen Fläche aufweist.

28. Vorrichtung nach einem der Ansprüche 1 bis 27,
dadurch **gekennzeichnet**, daß das Beleuchtungssystem so ausgebildet ist, daß die Anregungswellenlängen durchstimmbar sind.

29. Vorrichtung nach einem der Ansprüche 1 bis 28,
dadurch **gekennzeichnet,** daß in der Bildebene eine Videoeinheit (8) angeordnet ist.

30. Vorrichtung nach Anspruch 29,
dadurch **gekennzeichnet,** daß die Videoeinheit (8) einen CCD-Bildaufnehmer aufweist, und
daß die Gasentladungslampe (6) eine periodisch arbeitende Blitzentladungslampe ist, deren Blitzbelichtungsphase eine Steuer- und Auswerteeinheit (10) so steuert, daß die Blitzbelichtung ausschließlich in die Lichtintegrationsphase des CCD-Aufnehmers fällt.

31. Vorrichtung nach Anspruch 30,
dadurch **gekennzeichnet,** daß das Beleuchtungssystem zusätzlich eine kontinuierlich arbeitende Lichtquelle aufweist.

32. Vorrichtung nach einem der Ansprüche 29 bis 31,
dadurch **gekennzeichnet,** daß die Videoeinheit eine variable Belichtungseinstellung aufweist.

33. Vorrichtung nach einem der Ansprüche 29 bis 32,
dadurch **gekennzeichnet,** daß das Ausgangssignal der Videoeinheit an ein Bildverarzeitungssystem (10) angelegt ist.

34. Vorrichtung nach Anspruch 33,
dadurch **gekennzeichnet**, daß das Bildverarbeitungssystem einen Farbtonbereich im dargestellten Farbbild ausblendet.

35. Vorrichtung nach Anspruch 34,
dadurch **gekennzeichnet,** daß das Bildverarbeitungssystem bei einer RGB-Verarbeitung den Blau- und/oder Grünkanal zur Kontrastanhebung an- und abschaltet.

36. Vorrichtung nach Anspruch 34 oder 35,
dadurch **gekennzeichnet,** daß das Bildverarbeitungssystem den ausgeblendeten Farbtonbereich shutterartig in das dargestellte Farbbild einblendet.

37. Vorrichtung nach einem der Ansprüche 34 bis 36,
dadurch **gekennzeichnet,** daß das Bildverarbeitungssystem derart ausgebildet ist, daß es eine HSI-Transformation durchführt.

38. Vorrichtung nach einem der Ansprüche 34 bis 37,
dadurch **gekennzeichnet,** daß das Bildverarbeitungssystem derart ausgebildet ist, daß es zur Bestimmung eventueller Tumore den Kontrastwert an einzelnen Stellen des Bilden für die maximale Fluoreszenzwellenlänge berechnet.

39. Vorrichtung nach Anspruch 38,
dadurch **gekennzeichnet,** daß das Bildverarbeitungssysttem bei Verwendung von Delta-Aminolävulinsäure als Photosensibilisator das Kontrastverhäitnis für die Wellenlänge 630 nm zur Intensität in dem Bereich von maximal 50 nm berechnet, in dem das Gesamtsystem einen spektralen Transmissionsgrad von mehr als 5% aufweist.

40. Vorrichtung nach einem der Ansprüche 1 bis 39,
dadurch **gekennzeichnet**, daß die lichtzuführende Einheit und die bildgebende Einheit in ein Mikroskop integriert sind.

41. Vorrichtung nach Anspruch 40,
dadurch **gekennzeichnet,** daß das Mikroskop ein Mikroskop für neurochirurgische oder ophthalmologische Untersuchungen ist.

42. Vorrichtung nach einem der Ansprüche 1 bis 39,
dadurch **gekennzeichnet,** daß die lichtzuführende Einheit und die bildgebende Einheit in ein Endoskop integriert sind.

**43.** Vorrichtung nach Anspruch 42,
dadurch **gekennzeichnet,** daß die aktive lichtdurchlässige Gesamtquerschnittsfläche der lichtzuführenden Einheit 4 mm$^2$ nicht überschreitet.

**44.** Vorrichtung nach Anspruch 42 oder 43,
dadurch **gekennzeichnet,** daß die Lichtquelle eine an sich bekannte endoskopische Lichtquelle ist.

**45.** Vorrichtung nach Anspruch 44,
dadurch **gekennzeichnet,** daß die Leistungsaufnahme der Lichtquelle wenigstens 300 Watt beträgt.

**Claims**

**1.** Apparatus for "in vivo" diagnosis by means of a reaction in biologic tissue, which is induced by light or by inherent fluorescence by an endogenic or exogenic photo sensitiser, comprising

- an illuminating system including at least one light source with a lamp system (6) which generates incoherent light within the wave length range from at least 380 to 680 nm,
- a light feeder unit (2, 5, 21) which directs the light of said illuminating system onto the tissue region (7) to be diagnosed and/or for therapy, and
- an imaging, image detecting as well as image transmitting unit (31, 32, 4, 8) which images the light coming from said tissue region (7) into a proximal image plane (13),

wherein said spectral internal transmission factor $T_1(\lambda)$ of said light feeder unit is adapted to the spectrum of fluorescence stimulation of said photo sensitiser or said tissue, respectively, and the internal spectral transmission factor $Tb(\lambda)$ of said imaging unit is matched with the fluorescence spectrum of said photo sensitiser or said tissue, respectively, and
wherein the internal transmission factor of the overall system consisting of said light feeder unit and said imaging unit corresponds to more than 5 % only in a wave length range of 50 nm at maximum and is lower than 5 % in all other cases within the aforementioned wave length range.

**2.** Apparatus for "in vivo" diagnosis by means of a reaction in biologic tissue, which is induced by light or by inherent fluorescence by an endogenic or exogenic photo sensitiser, comprising

- an illuminating system including at least one light source
- a light feeder unit (2, 5, 21) which directs the light of said illuminating system onto the tissue region (7) to be diagnosed and/or for therapy, and
- an imaging, image detecting as well as image transmitting unit (31, 32, 4, 8) which images the light coming from said tissue region (7) into a proximal image plane (13),

wherein said spectral internal transmission factor $Ti_1(\lambda)$ of said light feeder unit comprises a first pass band adapted to the spectrum of fluorescence stimulation of said photo sensitiser or said tissue, respectively, and a second pass band whose wave lengths range between the wave lengths of the spectrum of fluorescence stimulation and the wave lengths of the fluorescence spectrum, and wherein the internal spectral transmission factor $Tb(\lambda)$ of said imaging unit comprises a first pass band matched with the fluorescence spectrum of said photo sensitiser or said tissue, respectively, and a second pass band ranging in the same wave length range as the second pass band of said light feeder unit, and
wherein the internal transmission factor of the overall system consisting of said light feeder unit and said imaging unit corresponds to more than 5 % only in said second pass band and is lower than 5 % in all other cases within the aforementioned wave length range.

**3.** Apparatus according to Claim 2,
**characterised** in that said first and second pass bands of said imaging unit are approximately complementary to each other.

**4.** Apparatus according to any of the Claims 1 to 3,
**characterised** in that said illuminating system comprises several light sources whereof one is a wideband light source having a lamp system and another one includes at least one laser system.

**5.** Apparatus according to Claim 4,
**characterised** in that all light sources of said illuminating system are coupled into said light feeder unit.

**6.** Apparatus according to any of the Claims 1 to 5,
**characterised** in that the transmission characteristics of said light transmitting and said imaging units are adjustable by means of one or several optical elements.

**7.** Apparatus according to Claim 6
**characterised** in that the transmission characteristics are so set that the overall intensity of the induced fluorescence light ranges in the same order as the overall intensity of the light fraction of said illuminating system which is reflected on the tissue region directly.

8. Apparatus according to Claim 6 or 7, **characterised** in that said optical elements are filter systems (9, 91, 92, 93) which are adapted to be inserted into the illumination or observation beam path, respectively.

9. Apparatus according to Claim 8, **characterised** in that said filter system adapted to be inserted into the illumination beam path and said filter system adapted to be inserted into the observation beam path display an approximately complementary filter characteristic.

10. Apparatus according to Claim 9 in combination with Claim 1, **characterised** in that said two complementary filter graphs intersect each other at an internal transmission factor of the individual systems of less than 50 %.

11. Apparatus according to any of the Claims 8 to 10, **characterised** in that the filter system in the illumination beam path includes at least two filters adapted to be inserted into the beam path, whereof one filter is a thermally stable interference filter (91) while the other filter is a thermally stable heat-absorbing filter (92).

12. Apparatus according to Claim 11, **characterised** in that with application of delta amino levulinic acid as photo sensitiser said thermally stable interference filter (91) displays the following transmission properties in the visible range:

$$T1 (\lambda = 380 - 430 \text{ nm}) > 50\%$$

$$T1 (\lambda = 610 - 650 \text{ nm}) < 1 \%.$$

13. Apparatus according to Claim 12, **characterised** in that the blocking factor of said thermally stable interference filter (91) is higher than 1000 at a wave length $\lambda$ of 630 nm, relative to the transmission maximum in the pass band at a wave length of approximately 407 nm.

14. Apparatus according to Claim 12 or 13 in combination with Claim 1, **characterised** in that said thermally stable interference filter (short-pass filter 91) displays the following characteristic:

$$T1(\lambda = 370 - 440 \text{ nm}) > 95 \%$$

$$T1(\lambda = 447 \text{ nm}) = 50 \%$$

$$T1(\lambda = 460 - 600 \text{ nm}) < 1 \%$$

$$T1(\lambda = 600 - 660 \text{ nm}) < 0.1 \%$$

$$T1(\lambda = 660 - 680 \text{ nm}) < 1 \%.$$

15. Apparatus according to Claim 12 or 13 in combination with Claim 2, **characterised** in that said thermally stable interference filter (short-pass filter 91) displays the following characteristic:

$$T1 (\lambda = 370 - 440 \text{ nm}) > 95 \%$$

$$T1(\lambda = 447 \text{ nm}) = 50 \%$$

$$T1(\lambda = 460 - 480 \text{ nm}) < 1 \%$$

$$T1(\lambda = 490 \text{ nm}) > 10 \%$$

$$T1(\lambda = 500 - 600 \text{ nm}) < 1 \%$$

$$T1(\lambda = 600 - 660 \text{ nm}) < 0.1 \%$$

$$T1 (\lambda = 660 - 680 \text{ nm}) < 1 \%.$$

16. Apparatus according to Claim 14 or 15. **characterised** in that the tolerance for the wave length at which the transmission corresponds to 50 % is $\pm$ 2 nm.

17. Apparatus according to any of the Claims 12 to 16, **characterised** in that said thermally stable heat-absorbing filter (92) displays the following characteristic:

$$T_1(\lambda = 370 - 440 \text{ nm}) > 95 \%$$

$$T_1(\lambda = 440 - 700 \text{ nm}) \approx 90 \%$$

$$T_1(\lambda = 700 \text{ nm}) = 50 \text{ \%}$$

$$T_1(\lambda = 720 - 1100 \text{ nm}) < 1 \text{ \%}.$$

18. Apparatus according to any of the Claims 9 to 17, **characterised** in that said thermally stable interference filter (91) is inserted into the illumination beam path only for performing the diagnostic operation.

19. Apparatus according to any of the Claims 1 or 3 to 18, **characterised** in that the internal transmission factor of the overall system is higher than 5 % only in the range between 430 and 460 nm and reaches a maximum value not exceeding the approximate level of 50 %, preferably roughly 15 % to 35 %, in this range.

20. Apparatus according to any of the Claims 8 to 19, **characterised** in that the removal of said filters (91, 92, 93) from the beam paths is possible only when such removal is released by a monitoring signal.

21. Apparatus according to any of the Claims 8 to 20, **characterised** in that the substrate material of the filters of said filter system is quartz or a thermally stable glass material.

22. Apparatus according to any of the Claims 1 to 21, **characterised** in that said light source (6) comprises at least a gas discharge tube.

23. Apparatus according to Claim 22, **characterised** in that said gas discharge tube is a xenon gas discharge tube.

24. Apparatus according to any of the Claims 1 to 23, **characterised** in that said light feeder unit comprises an optical guide (21) including at least a fibre having a numerical aperture exceeding 0.45.

25. Apparatus according to Claim 24, **characterised** in that the core material of said fibre consists of quartz and the envelope consists of a thermally stable material.

26. Apparatus according to Claim 24 or 25, **characterised** in that said gas discharge tube of said illuminating system has a focal spot with a diameter of less than 2 mm and an elliptic reflector having a numerical aperture for the light exit of more than 0.45.

27. Apparatus according to Claim 24 or 25, **characterised** in that said gas discharge tube of said illuminating system has a focal spot with a diameter of less than 2 mm and a parabolic reflector as well as a lens system as focussing unit with at least one non-spherical surface.

28. Apparatus according to any of the Claims 1 to 27, **characterised** in that said illuminating system is so configured that the stimulation wave lengths can be finely tuned.

29. Apparatus according to any of the Claims 1 to 28, **characterised** in that a video unit (8) is arranged in the image plane.

30. Apparatus according to Claim 29, **characterised** in that said video unit (8) comprises a CCD image pick-up means, and that said gas discharge tube (6) is a periodically operating flash discharge tube with a flash exposure phase controlling a controller and analyser unit (10) in such a way that the flash exposure takes place exclusively in the light integration phase of said CCD pick-up means.

31. Apparatus according to Claim 30, **characterised** in that said illuminating system comprises an additional continuously operating light source.

32. Apparatus according to any of the Claims 29 to 32, **characterised** in that said video unit comprises a variable exposure setting means.

33. Apparatus according to any of the Claims 29 to 32, **characterised** in that the output signal of said video unit is applied to an image processing system (10).

34. Apparatus according to Claim 33, **characterised** in that said image processing system masks out a hue range in the displayed multi-colour image.

35. Apparatus according to Claim 34, **characterised** in that in the case of RGB processing said image processing system turns the blue and/or green channel for increasing the contrast on and off.

36. Apparatus according to Claim 34 or 35, **characterised** in that said image processing system mixes the masked-out hue range into the displayed multi-colour image in a shutter-like manner.

37. Apparatus according to any of the Claims 34 to 36, **characterised** in that said image processing system is so configured that it implements an HSI transformation.

38. Apparatus according to any of the Claims 34 to 37,

**characterised** in that for the determination of possibly existing tumours said image processing system is so configured that it computes the contrast value at isolated positions of the image for the maximum fluorescence wave length.

39. Apparatus according to Claim 38,
**characterised** in that with application of delta amino levulinic acid as photo sensitiser said image processing system computes the contrast ratio for the wave length of 630 nm for the intensity in the range of 50 nm at maximum in which the overall system presents a spectral internal transmission factor of more than 5 %.

40. Apparatus according to any of the Claims 1 to 39,
**characterised** in that said light feeder unit and said imaging unit are integrated into a microscope.

41. Apparatus according to Claim 40,
**characterised** in that said microscope is a microscope for neuro surgical or ophthalmologic examinations.

42. Apparatus according to any of the Claims 1 to 39,
**characterised** in that said light feeder unit and said imaging unit are integrated into an endoscope.

43. Apparatus according to Claim 42,
**characterised** in that the active translucent total cross-sectional surface of said light feeder unit does not exceed 4 mm$^2$.

44. Apparatus according to Claim 42 or 43,
**characterised** in that said light source is an endoscopic light source known per se.

45. Apparatus according to Claim 44,
**characterised** in that the power consumption of said light source amounts to at least 300 Watt.


**Revendications**

1. Dispositif de diagnostic "in vivo" moyennant une réaction dans le tissu biologique, qui est induite par lumière ou par auto-fluorescence moyennant un photo-sensibilisateur endogène ou exogène, comprenant

   - un système d'éclairage comprenant au moins une source lumineuse à un système de lampes (6), qui engendre de la lumière non cohérente au-dedans de la gamme de longueurs d'ondes d'au moins 380 à 680 nm,
   - une unité d'amenée de lumière (2, 5, 21), qui oriente la lumière dudit système d'éclairage sur la zone de tissu (7) à diagnostiquer et/ou pour la thérapie, et
   - une unité d'imagerie, une unité détecteur d'image ainsi qu'une unité de transmission d'image (31, 32, 4, 8), qui produit l'image de la lumière, qui vient de ladite zone de tissu (7), dans un plan d'image proximal (13),

dans lequel ladite transmittance interne spectrale $T_1(\lambda)$ de ladite unité d'amenée de lumière est adaptée au spectre de stimulation de fluorescence dudit photo-sensibilisateur ou respectivement dudit tissu, et la transmittance interne spectrale $Tb(\lambda)$ de ladite unité d'imagerie est adaptée au spectre de fluorescence dudit photo-sensibilisateur ou respectivement dudit tissu, et
dans lequel la transmittance interne du système total, qui est composé de ladite unité d'amenée de lumière et ladite unité d'imagerie, ne correspond à un taux supérieure à 5 % qu'au-dedans d'une gamme d'ondes de 50 nm au maximum, en étant inférieure à 5 % dans tous les autres cas au-dedans de ladite gamme d'ondes susmentionnée.

2. Dispositif de diagnostic "in vivo" moyennant une réaction dans le tissu biologique, qui est induite par lumière ou par auto-fluorescence moyennant un photo-sensibilisateur endogène ou exogène, comprenant

   - un système d'éclairage comprenant au moins une source lumineuse à un système de lampes (6), qui engendre de la lumière non cohérente au-dedans de la gamme de longueurs d'ondes d'au moins 380 à 680 nm,
   - une unité d'amenée de lumière (2, 5, 21), qui oriente la lumière dudit système d'éclairage sur la zone de tissu (7) à diagnostiquer et/ou pour la thérapie, et
   - une unité d'imagerie, une unité détecteur d'image ainsi qu'une unité de transmission d'image (31, 32, 4, 8), qui produit l'image de la lumière, qui vient de ladite zone de tissu (7), dans un plan d'image proximal (13),

dans lequel ladite transmittance interne spectrale $Ti_1(\lambda)$ de ladite unité d'amenée de lumière comprend une première bande passante adaptée au spectre de stimulation de fluorescence dudit photo-sensibilisateur ou respectivement dudit tissu, et une deuxième bande passante, dont les longueurs d'ondes varient entre les longueurs d'ondes du spectre de stimulation de fluorescence et les longueurs d'ondes du spectre de fluorescence, et dans lequel la transmittance interne spectrale $Tb(\lambda)$ de ladite unité d'imagerie comprend une première bande passante adaptée au spectre de fluorescence dudit photo-sensibilisateur ou respectivement dudit tissu, et une deuxième bande passante qui varient au-dedans de la même gamme d'ondes que la deuxième bande passante de ladite unité d'amenée de lumière, et

dans lequel la transmittance interne du système total, qui est composé de ladite unité d'amenée de lumière et ladite unité d'imagerie, ne correspond à une valeur supérieure à 5 % qu'au-dedans de ladite deuxième bande passante, en étant inférieure à 5 % dans tous les autres cas au-dedans de ladite gamme d'ondes susmentionnée.

**3.** Dispositif selon la revendication 2,
**caractérisé** en ce que lesdits première et deuxième bandes passantes de ladite unité d'imagerie sont complémentaire environ l'une à l'autre.

**4.** Dispositif selon une quelconque des revendications 1 à 3,
**caractérisé** en ce que ledit système d'éclairage comprend plusieurs source lumineuses, dont une est une source lumineuse à large bande à un système de lampes, pendant que l'autre comprend au moins un système laser.

**5.** Dispositif selon la revendication 4,
**caractérisé** en ce que tous les source lumineuses dudit système d'éclairage sont couplées dans ladite unité d'amenée de lumière.

**6.** Dispositif selon une quelconque des revendications 1 à 5,
**caractérisé** en ce que les caractéristiques de transmission desdites unités de transmission de lumière et d'imagerie sont réglables moyennant un ou plusieurs éléments optiques.

**7.** Dispositif selon la revendication 6
**caractérisé** en ce que les caractéristiques de transmission sont ajustées de façon, que l'intensité globale de la lumière fluorescente induite système d'éclairage, qui est réfléchie sur la zone de tissu directement.

**8.** Dispositif selon la revendication 6 ou 7,
**caractérisé** en ce que lesdits éléments optiques sont des systèmes à filtres (9, 91, 92, 93) aptes à être insérés dans la marche des rayons d'éclairage ou respectivement dans la marche des rayons de la lumière d'observation.

**9.** Dispositif selon la revendication 8,
**caractérisé** en ce que ledit système à filtres apte à être introduit dans la marche de rayons de la lumière d'éclairage et ledit système à filtres apte à être inséré dans la marche des rayons de la lumière d'observation présentent une caractéristique de filtre complémentaire environ.

**10.** Dispositif selon la revendication 9 en combinaison avec la revendication 1,
**caractérisé** en ce que les plots desdits deux filtres se croisent à une transmittance interne des systèmes individuels, qui est inférieure à 50 %.

**11.** Dispositif selon une quelconque des revendications 8 à 10,
**caractérisé** en ce que ledit système à filtres dans la marche des rayons de la lumière d'éclairage comprend au moins deux filtres aptes à être insérés dans la marche des rayons, dont l'un filtre est un filtre d'interférence thermostable (91) pendant que l'autre filtre est un filtre anticalorique thermostable (92).

**12.** Dispositif selon la revendication 11,
**caractérisé** en ce qu'à l'application de l'aminoacide lévulique delta en tant que le photo-sensibilisateur, ledit filtre d'interférence thermostable (91) présente les caractéristiques de transmission suivantes dans la gamme lumineuse:

$$T1 \ (\lambda = 380 - 430 \ nm) > 50 \ \%$$

$$T1 \ (\lambda = 610 - 650 \ nm) < 1 \ \%.$$

**13.** Dispositif selon la revendication 12,
**caractérisé** en ce que le facteur de blocage dudit filtre d'interférence thermostable (91) est supérieur à 1000 à une longueur d'onde $\lambda$ de 630 nm, relative à la transmission maximale dans la bande passante à une longueur d'onde de 407 nm environ.

**14.** Dispositif selon la revendication 12 ou 13 en combinaison avec la revendication 1,
**caractérisé** en ce que ledit filtre d'interférence thermostable (filtre à courte bande passante 91) présente la caractéristique suivante:

$$T1 \ (\lambda = 370 - 440 \ nm) > 95 \ \%$$

$$T1 \ (\lambda = 447 \ nm) = 50 \ \%$$

$$T1 \ (\lambda = 460 - 600 \ nm) < 1 \ \%$$

$$T1 \ (\lambda = 600 - 660 \ nm) < 0,1 \ \%$$

$$T1 \ (\lambda = 660 - 680 \ nm) < 1 \ \%.$$

**15.** Dispositif selon la revendication 12 ou 13 en combinaison avec la revendication 2,
**caractérisé** en ce que ledit filtre d'interférence thermostable (filtre à courte bande passante 91) présente la caractéristique suivante:

$$T1 (\lambda = 370 - 440 \text{ nm}) > 95 \text{ \%}$$

$$T1 (\lambda = 447 \text{ nm}) = 50 \text{ \%}$$

$$T1 (\lambda = 460 - 480 \text{ nm}) < 1 \text{ \%}$$

$$T1 (\lambda = 490 \text{ nm}) > 10 \text{ \%}$$

$$T1 (\lambda = 500 - 600 \text{ nm}) < 1 \text{ \%}$$

$$T1 (\lambda = 600 - 660 \text{ nm}) < 0,1 \text{ \%}$$

$$T1 (\lambda = 660 - 680 \text{ nm}) < 1 \text{ \%}.$$

16. Dispositif selon la revendication 14 ou 15.
**caractérisé** en ce que la tolérance pour la longueur d'onde, à laquelle la transmission correspond à 50 %, est $\pm$ 2 nm.

17. Dispositif selon une quelconque des revendications 12 à 16,
**caractérisé** en ce que ledit filtre anticalorique thermostable (92) présente la caractéristique suivante:

$$T_1 (\lambda = 370 - 440 \text{ nm}) > 95 \text{ \%}$$

$$T_1 (\lambda = 440 - 700 \text{ nm}) \approx 90 \text{ \%}$$

$$T_1 (\lambda = 700 \text{ nm}) = 50 \text{ \%}$$

$$T_1 (\lambda = 720 - 1100 \text{ nm}) < 1 \text{ \%}.$$

18. Dispositif selon une quelconque des revendications 9 à 17,
**caractérisé** en ce que ledit filtre d'interférence thermostable (91) est inséré dans la marche des rayons de la lumière d'éclairage seulement pour la réalisation d'une opération de diagnostic.

19. Dispositif selon une quelconque des revendications 1 ou 3 à 18,
**caractérisé** en ce que la transmittance interne du système total n'est supérieure à 5 % qu'au-dedans de la gamme entre 430 et 460 nm et atteigne une valeur maximale égale ou inférieure à un taux de 50 % environ, de préférence de 15 % à 35 % environ, au-dedans de cette gamme.

20. Dispositif selon une quelconque des revendications 8 à 19,
**caractérisé** en ce que le mouvement desdits filtres (91, 92, 93) en dehors des marches des rayons n'est possible que quand un tel mouvement est déclenché par un signal de surveillance.

21. Dispositif selon une quelconque des revendications 8 à 20,
**caractérisé** en ce que la matériau de substrat des filtres dudit système à filtres est quartz ou un matériau vitreux thermostable.

22. Dispositif selon une quelconque des revendications 1 à 21,
**caractérisé** en ce que ladite source lumineuse (6) comprend au moins un tube à gaz lumineux.

23. Dispositif selon la revendication 22,
**caractérisé** en ce que ledit tube à gaz lumineux est un tube à gaz xénon lumineux.

24. Dispositif selon une quelconque des revendications 1 à 23,
**caractérisé** en ce que ladite unité d'amenée de lumière comprend un guide de lumière (21) qui renferme au moins une fibre dont l'ouverture numérique est supérieure à 0,45.

25. Dispositif selon la revendication 24,
**caractérisé** en ce que le matériau de l'âme de ladite fibre est faite en quartz pendant que l'enveloppe est faite en un matériau thermostable.

26. Dispositif selon la revendication 24 or 25,
**caractérisé** en ce que ledit tube à gaz lumineux dudit système d'éclairage a un foyer d'un diamètre inférieur à 2 mm, et un réflecteur elliptique à une ouverture numérique pour la sortie de lumière, qui est supérieure à 0,45.

27. Dispositif selon la revendication 24 ou 25,
**caractérisé** en ce que ledit tube à gaz lumineux dudit système d'éclairage a un foyer d'un diamètre inférieur à 2 mm, et un réflecteur parabolique ainsi qu'un système à lentilles comme l'unité de focalisation, à au moins une aire non sphérique.

28. Dispositif selon une quelconque des revendications 1 à 27,
**caractérisé** en ce que ledit système d'éclairage est configuré de façon qu'une commande à fréquence variable des longueurs d'ondes de stimulation soit possible.

29. Dispositif selon une quelconque des revendications 1 à 28,
    **caractérisé** en ce qu'une unité vidéo (8) est disposée dans le plan d'image.

30. Dispositif selon la revendication 29,
    **caractérisé** en ce que ladite unité vidéo (8) comprend un moyen de prise de vues CCD, et en ce que ledit tube à gaz lumineux (6) est un tube à fonctionnement flash périodique, à une phase de pose flash, qui commande une unité de commande et analyseur (10) d'une manière, que la pose flash se fasse exclusivement au cours de la phase d'intégration de lumière dudit moyen de prise de vues CCD.

31. Dispositif selon la revendication 30,
    **caractérisé** en ce que ledit système d'éclairage comprend une source lumineuse additionnelle à fonctionnement continu.

32. Dispositif selon une quelconque des revendications 29 à 32,
    **caractérisé** en ce que ladite unité vidéo unit comprend un moyen variable d'ajustage de pose.

33. Dispositif selon une quelconque des revendications 29 à 32,
    **caractérisé** en ce que le signal de sortie de ladite unité vidéo est appliqué à un système de traitement d'images (10).

34. Dispositif selon la revendication 33,
    **caractérisé** en ce que ledit système de traitement d'images supprime une gamme de tonalité dans l'image en couleurs visualisée.

35. Dispositif selon la revendication 34,
    **caractérisé** en ce qu'au cas d'un traitement du type RGB dans ledit système de traitement d'images active et désactive la voie bleue et/ou verte afin d'augmenter le contraste.

36. Dispositif selon la revendication 34 or 35,
    **caractérisé** en ce que ledit système de traitement d'images mélange la gamme de tonalités supprimée dans l'image en couleurs visualisée de la façon d'un obturateur.

37. Dispositif selon une quelconque des revendications 34 à 36,
    **caractérisé** en ce que ledit système de traitement d'images est configuré de façon qu'il réalise une transformation dite HSI.

38. Dispositif selon une quelconque des revendications 34 à 37,
    **caractérisé** en ce que pour la détection de tumeurs éventuellement présents, ledit système de traitement d'images est configuré de façon qu'il calcule la valeur de contraste aux positions isolées de l'image pour la longueur d'onde maximale de fluorescence.

39. Dispositif selon la revendication 38,
    **caractérisé** en ce qu'au cas de l'emploi de aminoacide lévulique delta comme le photo-sensibilisateur, ledit système de traitement d'images calcule le rapport de contraste pour la longueur d'onde de 630 nm pour l'intensité dans la gamme de 50 nm au maximum, dans laquelle le système total présente une transmittance interne spectrale supérieure à 5 %.

40. Dispositif selon une quelconque des revendications 1 à 39,
    **caractérisé** en ce que ladite unité d'amenée de lumière et ladite unité d'imagerie sont intégrées dans un microscope.

41. Dispositif selon la revendication 40,
    **caractérisé** en ce que ledit microscope est un microscope pour le diagnostic en neurochirurgie ou ophtalmologie.

42. Dispositif selon une quelconque des revendications 1 à 39,
    **caractérisé** en ce que ladite unité d'amenée de lumière et ladite unité d'imagerie sont intégrées dans un endoscope.

43. Dispositif selon la revendication 42,
    **caractérisé** en ce que l'aire active totale transparente en coupe transversale de ladite unité d'amenée de lumière est égale ou inférieure à 4 mm$^2$.

44. Dispositif selon la revendication 42 ou 43,
    **caractérisé** en ce que ladite source lumineuse est une source lumineuse endoscopique connue en soi.

45. Dispositif selon la revendication 44,
    **caractérisé** en ce que la puissance absorbée de ladite source lumineuse correspond à au moins 300 Watt.

Fig.1

Wellenlänge [nm]

Fluoreszenzspektrum

635

Transmission des Beobachtungssystems

Transmission des Beleuchtungssystems (Anregungsspektrum)

Fig. 2a

410

380

780

100

0

Transmission [%]

Fig. 26

EP 0 861 044 B1

Fig. 3a

Fig. 36